(19) Europäisches Patentamt
European Patent Office
Office européen des brevets

(11) Veröffentlichungsnummer: **0 289 850**
**A2**

(12) # EUROPÄISCHE PATENTANMELDUNG

(21) Anmeldenummer: **88106190.7**

(22) Anmeldetag: **19.04.88**

(51) Int. Cl.4: **C07D 207/325 , C07D 207/34 , C07D 405/12 , C07D 413/10 , A61K 31/40**

(30) Priorität: **30.04.87 DE 3714485**

(43) Veröffentlichungstag der Anmeldung:
**09.11.88 Patentblatt 88/45**

(84) Benannte Vertragsstaaten:
**AT BE CH DE ES FR GB GR IT LI NL SE**

(71) Anmelder: **BAYER AG**
**Konzernverwaltung RP Patentabteilung**
**D-5090 Leverkusen 1 Bayerwerk(DE)**

(72) Erfinder: **Wollweber, Hartmund, Dr.**
**In der Birken 73**
**D-5600 Wuppertal 1(DE)**
Erfinder: **Stoltefuss, Jürgen, DI.**
**Parkstrasse 20**
**D-5657 Haan(DE)**
Erfinder: **Berschauer, Friedrich, Dr.**
**Claudiusweg 9**
**D-5600 Wuppertal 1(DE)**
Erfinder: **de Jong, Anno, Dr.**
**Stockmannsmühle 46**
**D-5600 Wuppertal 1(DE)**
Erfinder: **Scheer, Martin, Dr.**
**Herberts-Katernberg 7**
**D-5600 Wuppertal 1(DE)**

(54) **Pyrrolophenylalkanolamine und ihre Derivate, Verfahren zu ihrer Herstellung und ihre Verwendung als Leistungsförderer.**

(57) Die vorliegende Erfindung betrifft Pyrrolophenylalkanolamine der Formel I

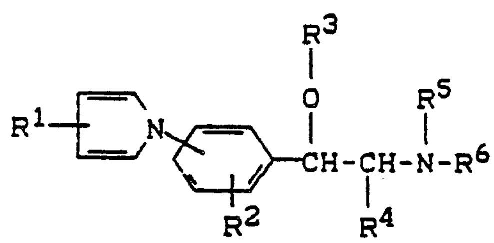

I

in welcher
$R^1$ bis $R^6$ die in der Beschreibung angegebene Bedeutung besitzen.
Verfahren zu ihrer Herstellung und neue Zwischenprodukte zur Durchführung dieser Verfahren.
Die Verbindungen der Formel I sowie ihre physiologisch verträglichen Salze besitzen leistungsfördernde Wirkung bei Tieren, insbesondere eine Wirkung zur Verschiebung des Fleisch/Fett-Verhältnisses zugunsten von

EP 0 289 850 A2

Fleisch.

## Pyrrolophenylalkanolamine und ihre Derivate, Verfahren zu ihrer Herstellung und ihre Verwendung als Leistungsförderer

Die vorliegende Erfindung betrifft neue Pyrrolophenylalkanolamine, ihre Derivate, Verfahren zu ihrer Herstellung sowie ihre Verwendung als Leistungsförderer bei Tieren.

Es ist bekannt, daß substituierte Phenylethanolamine das Wachstum von Tieren fördernde Eigenschaften besitzen (EP-OS 26 298, EP-OS 49 728). Die leistungsfördernden Eigenschaften der bekannten Verbindungen sind jedoch nicht immer voll befriedigend.

Die vorliegende Erfindung betrifft

1. die neuen Pyrrolophenylalkanolamine der Formel I und ihre Derivate

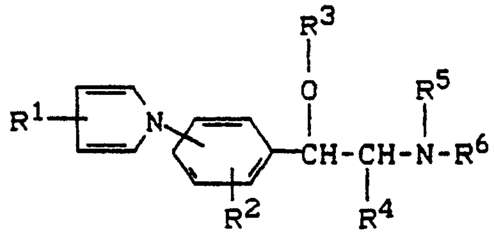

I

in welcher

$R^1$ für einen oder mehrere gleiche oder verschiedene Reste aus der Gruppe Wasserstoff, Halogen, Cyano, Formyl, Nitro, Carboxyl, Carbalkoxyalkyl, Alkoxycarboxyalkyl, Alkylcarbonyl, Alkoxycarbonyl, Aminocarbonyl, Alkylaminocarbonyl, Dialkylaminocarbonyl, Alkyl, Alkoxy, Alkenoxy, Halogenalkyl, Halogenalkoxy, Alkylthio, Halogenalkylthio, Hydroxyalkyl, Cyanoalkyl, Alkoxyalkyl, Alkylthioalkyl, Alkylcarbonylalkoxy steht,

$R^2$ für einen oder mehrere gleiche oder verschiedene Reste aus der Gruppe Wasserstoff, Hydroxy, Halogen, Cyano, Nitro, Alkyl, Alkoxy, Alkylthio, Halogenalkyl, Halogenalkoxy, Halogenalkylthio, Cyanoalkyl, Alkoxyalkyl, Hydroxyalkyl, Alkoxycarbonyl, Aminocarbonyl, Mono-, Dialkylaminocarbonyl steht,

$R^3$ für Wasserstoff, Acyl oder Trialkylsilyl steht,

$R^4$ für Wasserstoff oder Alkyl steht,

$R^5$ für Wasserstoff oder gemeinsam mit $R^3$ für folgende Reste steht,

$-\underset{\substack{| \\ R^7}}{CH}-$; $-\underset{\substack{\| \\ O}}{C}-$

wobei $R^7$ für Wasserstoff oder Alkyl steht,

$R^6$ für verzweigtes oder cyclisches Alkyl steht, die gegebenenfalls substituiert sind.

2. Verfahren zur Herstellung der neuen Pyrrolophenylalkanolamine der Formel I und ihrer Derivate

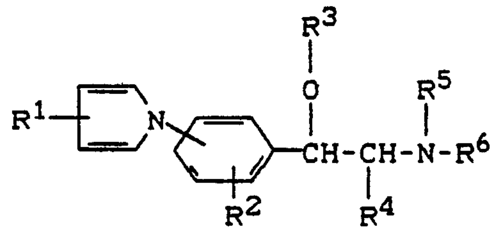

I

in welcher

$R^1$ für einen oder mehrere gleiche oder verschiedene Reste aus der Gruppe Wasserstoff, Halogen, Cyano, Formyl, Nitro, Carboxyl, Carbalkoxyalkyl, Alkoxycarboxyalkyl, Alkylcarbonyl, Alkoxycarbonyl, Aminocarbonyl, Alkylaminocarbonyl, Dialkylaminocarbonyl, Alkyl, Alkoxy, Alkenoxy, Halogenalkyl, Halogenalkoxy, Alkylthio, Halogenalkylthio, Hydroxyalkyl, Cyanoalkyl, Alkoxyalkyl, Alkylthioalkyl, Alkylcarbonylalkoxy steht,

$R^2$ für einen oder mehrere gleiche oder verschiedene Reste aus der Gruppe Wasserstoff, Hydroxy, Halogen, Cyano, Nitro, Alkyl, Alkoxy, Alkylthio, Halogenalkyl, Halogenalkoxy, Halogenalkylthio, Cyanoalkyl, Alkoxyalkyl, Hydroxyalkyl, Alkoxycarbonyl, Aminocarbonyl, Mono-, Dialkylaminocarbonyl steht,

$R^3$ für Wasserstoff, Acyl oder Trialkylsilyl steht,

$R^4$ für Wasserstoff oder Alkyl steht,

$R^5$ für Wasserstoff oder gemeinsam mit $R^3$ für folgende Reste steht,

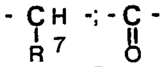

wobei $R^7$ für Wasserstoff oder Alkyl steht,
$R^6$ für verzweigtes oder cyclisches Alkyl steht, die gegebenenfalls substituiert sind, dadurch gekennzeichnet, daß man Verbindungen der Formel II

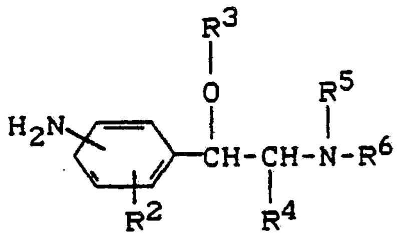

II

in welcher
$R^2$, $R^3$, $R^4$, $R^5$, $R^6$ die oben angegebene Bedeutung haben,
a) mit 1,4-Dicarbonylverbindungen der Formel III

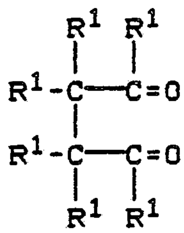

III

oder deren Monoacetale, Diacetale oder cyclische Acetale der Formel IIa, IIIb oder IIIc

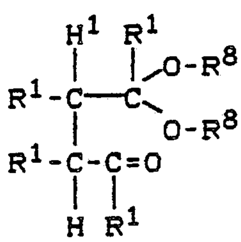

;

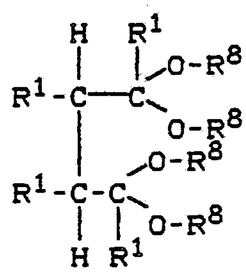

IIIa

IIIb

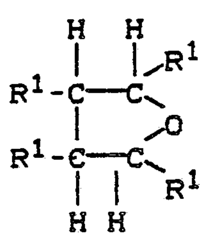

IIIc

in welchen
$R^1$ die oben angegebene Bedeutung hat,
$R^8$ für Wasserstoff, Alkyl oder Aryl steht,
umsetzt oder

b) mit Dihalogenallenen der Formel IV

$Hal-CH=C=CH-CH_2-Hal$ IV

in welcher
Hal für Halogen steht,
umsetzt oder

c) mit Epoxybutanen der Formel V

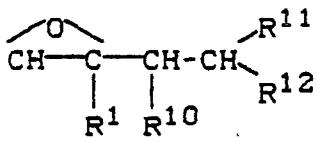

V

in welcher
$R^1$ die oben angegebene Bedeutung hat,
$R^{10}$ für Wasserstoff oder Brom steht,
$R^{11}$ für Wasserstoff und
$R^{12}$ für Brom steht oder
$R^{11}$ und $R^{12}$ für Alkoxy oder Aryloxy stehen,
umsetzt oder

d) mit Halogencrotonaldehyd der Formel VI

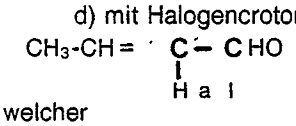

VI

in welcher
Hal für Halogen steht,
umsetzt oder

e) indem man Verbindungen der Formel VII

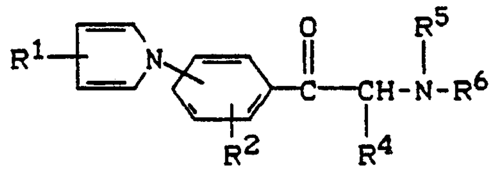

VII

in welcher
$R^1$, $R^2$, $R^4$, $R^5$, $R^6$ die oben angegebene Bedeutung haben,
reduziert, oder

f) indem man Verbindungen der Formel VIII

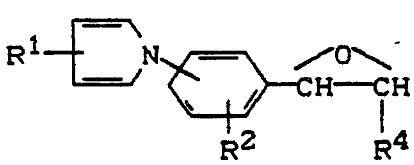

VIII

in welcher
$R^1$, $R^2$, $R^4$ die oben angegebene Bedeutung haben,
mit Aminen der Formel IX

$HNR^5R^6$ IX

in welcher
$R^5$ und $R^6$ die oben angegebene Bedeutung haben,
umsetzt, oder

g) indem man Verbindungen der Formel X

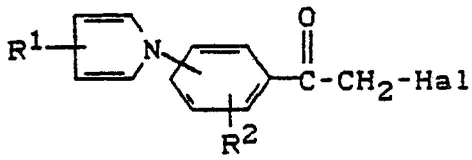

X

in welcher
$R^1$ und $R^2$ die oben angegebene Bedeutung haben und
Hal für Halogen steht,
mit Aminen der Formel IX umsetzt und anschließend die Carbonylgruppe reduziert, oder

h) indem man Verbindungen der Formel XI

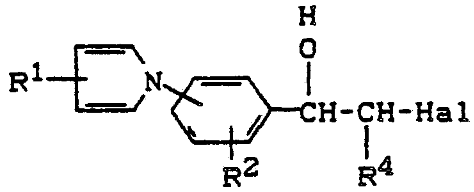

XI

in welcher
$R^1$, $R^2$, $R^4$ die oben angegebene Bedeutung haben,
Hal für Halogen steht,
mit Aminen der Formel IX umsetzt, oder

i) indem man für den Fall, daß in Formel I der Rest $R^5$ für Wasserstoff steht, Verbindungen der Formel XII.

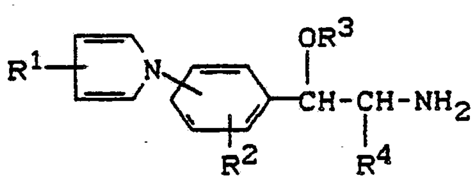

XII

in welcher
$R^1$ bis $R^4$ die oben angegebene Bedeutung haben,
mit Ketonen der Formel XIII

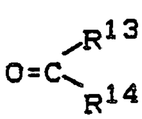

XIII

in welcher
$R^{13}$ für gegebenenfalls substituiertes Alkyl oder Cycloalkyl steht,
$R^{14}$ für gegebenenfalls substituiertes Alkyl, Cycloalkyl, Heterocyclyl steht,
$R^{13}$ und $R^{14}$ stehen gemeinsam mit dem angrenzenden C-Atom für einen gegebenenfalls substituierten aliphatischen Ring,
unter reduzierenden Bedingungen umsetzt, oder

j) indem man Verbindungen der Formel XIV

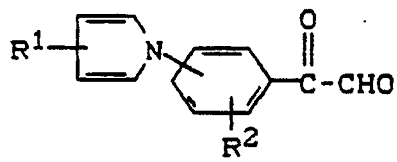

XIV

in welcher
$R^1$ und $R^2$ die oben angegebenen Bedeutungen haben,
mit Aminen der Formel IX unter reduzierenden Bedingungen umsetzt, oder

k) indem man für den Fall, daß in Formel I $R^3$ und $R^4$ für Wasserstoff stehen, Verbindungen der Formel XV

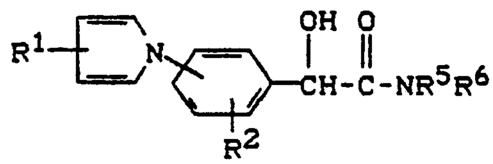

XV

in welcher
$R^1$, $R^2$, $R^5$, $R^6$ die oben angegebenen Bedeutungen haben,
reduziert, oder

l) indem man für den Fall, daß $R^3$ für Acyl steht, Verbindungen der Formel I in welcher $R^3$ für Wasserstoff steht, mit Acylierungsmitteln in Gegenwart von Basen umsetzt, oder

m) indem man für den Fall, daß $R^3$ für Trialkylsilyl steht, Verbindungen der Formel I in welcher $R^3$ für Wasserstoff steht,
mit Silylierungsmitteln der Formel XVI

$Z-Si(R^{15})_3$ XVI

in welcher
Z für Halogen, CN, $OSO_2-CF_3$, $O-Si(Alkyl)_3$ oder $O-SO_2-OSi(Alkyl)_3$ steht und
$R^{15}$ für gleiche oder verschiedene Alkylreste steht,
umsetzt, oder

n) indem man für den Fall, daß $R^3$ und $R^5$ gemeinsam für den Rest $-\underset{\underset{O}{\|}}{C}-$ stehen, Verbindungen der Formel I in welcher $R^3$ und $R^5$ für Wasserstoff stehen, mit Phosgen oder Phosgen abspaltenden Agentien umsetzt, oder

o) indem man für den Fall, daß $R^3$ und $R^5$ gemeinsam für den Rest $-\underset{\underset{R^7}{|}}{C}H-$ stehen, Verbindungen der Formel I in welcher $R^3$ und $R^5$ für Wasserstoff stehen, mit Aldehyden der Formel XVII

$R^7-\underset{\underset{H}{|}}{C}=O$ XVII

in welcher
$R^7$ die oben angegebene Bedeutung hat,
in Gegenwart wasserentziehender Mittel oder unter wasserentziehenden Reaktionsbedingungen umsetzt, oder

p) indem man Verbindungen der Formel XII, in der $R^1$ - $R^4$ die oben angegebene Bedeutung haben, mit Halogenverbindungen der Formel XXXVIII

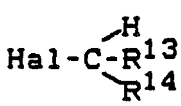

XXXVIII

in welcher
$R^{13}$ und $R^{14}$ die bei Verfahren 2i, bei den Verbindungen der Formel XIII angegebenen Bedeutungen haben und
Hal für Chlor oder Brom steht,

umsetzt.

3. Neue Verbindungen der Formel VII

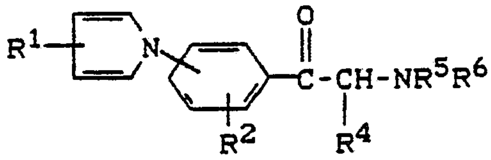

VII

in welcher

$R^1$, $R^2$, $R^4$ bis $R^6$ die bei den Verbindungen der Formel I angegebenen Bedeutungen haben.

4. Verfahren zur Herstellung der Verbindungen der Formel VII, dadurch gekennzeichnet, daß man wie bei Verfahren 2 g) in der ersten Stufe beschrieben, Halogenacetophenone der Formel X

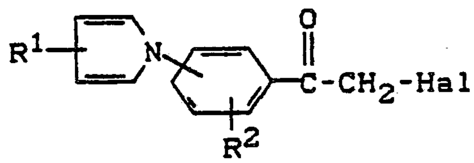

X

in welcher

$R^1$, $R^2$ die bei den Verbindungen der Formel I angegebenen Bedeutungen haben und

Hal für Halogen steht,

mit Aminen der Formel IX umsetzt, oder indem man Verbindungen der Formel XX

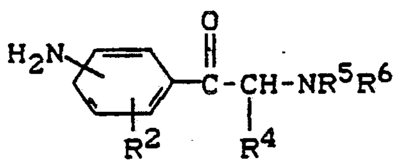

XX

in welcher

$R^2$, $R^4$ bis $R^6$ die bei den Verbindungen der Formel I angegebene Bedeutung besitzen und $R^5$ und $R^6$ nicht beide für Wasserstoff stehen,

analog zu den bei Verfahren 2 a-d) genannten Reaktionen umsetzt.

5. Neue Verbindungen der Formel VIII

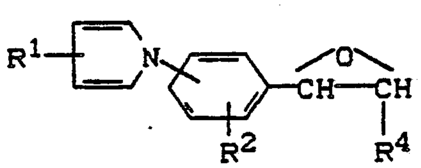

VIII

in welcher

$R^1$, $R^2$, $R^4$ die bei den Verbindungen der Formel I angegebenen Bedeutungen besitzen.

6. Verfahren zur Herstellung der Verbindungen der Formel VIII, dadurch gekennzeichnet, daß man Verbindungen der Formel XI

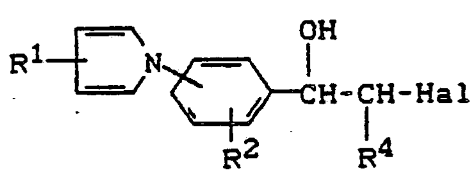

XI

in welcher
$R^1$, $R^2$, $R^4$ die bei den Verbindungen der Formel I angegebenen Bedeutungen haben und
Hal für Halogen steht,
mit Basen umsetzt oder daß man Verbindungen der Formel XVIII

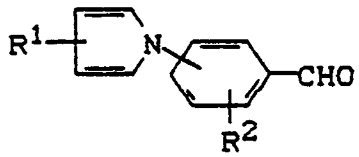

XVIII

in welcher
$R^1$ und $R^2$ die bei den Verbindungen der Formel I angegebenen Bedeutungen haben,
mit Methylengruppen-übertragenden Reagenzien in Gegenwart von Basen umsetzt,
oder indem man Verbindungen der Formel XXI

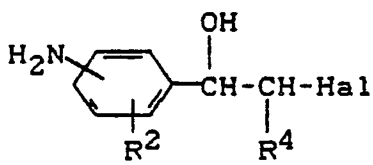

XXI

in welcher
$R^2$ und $R^4$ die bei den Verbindungen der Formel I genannten Bedeutungen besitzen und
Hal für Halogen steht
analog zu den bei Verfahren 2 a-d) genannten Reaktionen umsetzt.

7. Neue Verbindungen der Formel X

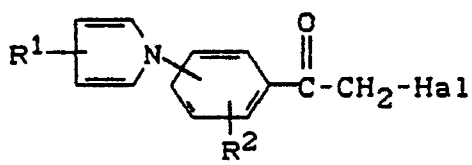

X

in welcher
$R^1$ und $R^2$ die bei den Verbindungen der Formel I angegebenen Bedeutungen besitzen und
Hal für Halogen steht.

8. Verfahren zur Herstellung der Verbindungen der Formel X, dadurch gekennzeichnet, daß man Acetophenone der Formel XIX

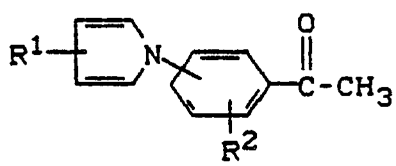

XIX

in welcher
$R^1$ und $R^2$ die bei den Verbindungen der Formel I angegebenen Bedeutungen haben,
mit Halogen oder Kupferhalogeniden $Cu(Halogen)_2$ oder N-Halogensuccinimiden umsetzt, oder indem man Verbindungen der Formel XXII

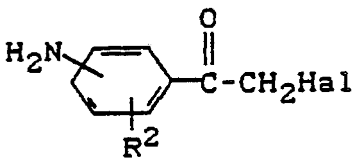

XXII

in welcher
$R^2$ die bei den Verbindungen der Formel I angegebenen Bedeutungen besitzt und
Hal für Halogen steht
analog zu den bei Verfahren 2 a-d) genannten Reaktionen umsetzt.

9. Neue Verbindungen der Formel XI

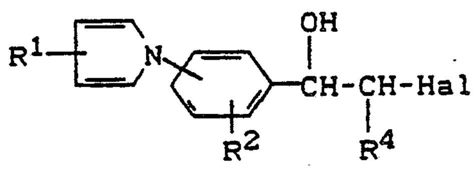

XI

in welcher
$R^1$, $R^2$ die bei den Verbindungen der Formel I angegebenen Bedeutungen besitzen,
Hal für Halogen steht und
$R^4$ für Wasserstoff steht.

10. Verfahren zur Herstellung der Verbindungen der Formel XI, dadurch gekennzeichnet, daß man Verbindungen der Formel X

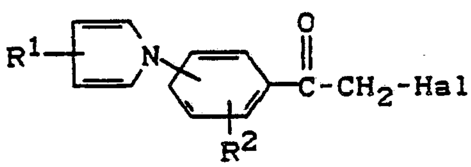

X

in welcher
$R^1$ und $R^2$ die bei den Verbindungen der Formel I angegebenen Bedeutungen haben und
Hal für Halogen steht,
reduziert oder, indem man Verbindungen der Formel XXIII

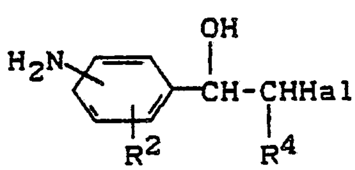

XXIII

in welcher
$R^2$ und $R^4$ die bei den Verbindungen der Formel I angegebene Bedeutung haben und
Hal für Halogen steht,
analog zu den bei Verfahren 2 a-d) genannten Reaktionen umsetzt.

11. Neue Verbindungen der Formel XII

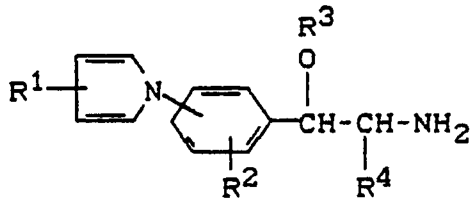

XII

in welcher
$R^1$ bis $R^4$ die bei den Verbindungen der Formel I angegebenen Bedeutungen besitzen.

12. Verfahren zur Herstellung der Verbindungen der Formel XII, dadurch gekennzeichnet, daß man Nitroverbindungen der Formel XXIV

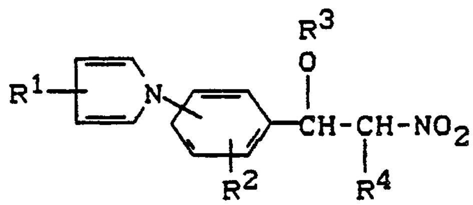

XXIV

in welcher
$R^1$ bis $R^4$ die bei den Verbindungen der Formel I angegebenen Bedeutungen haben,
reduziert oder, daß man Verbindungen der Formel XXV

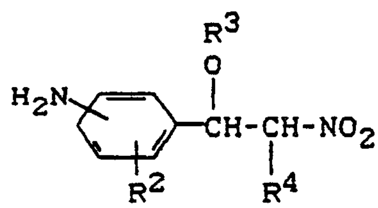

XXV

in welcher
$R^2$ bis $R^4$ die bei den Verbindungen der Formel I angegebenen Bedeutungen haben
analog zu den bei Verfahren 2 a-d) genannten Reaktionen umsetzt und anschließend reduziert.

13. Neue Verbindungen der Formel XIV

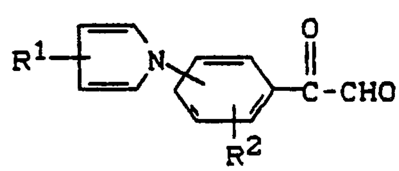

XIV

in welcher
$R^1$ und $R^2$ die bei den Verbindungen der Formel I angegebenen Bedeutungen besitzen.

14. Verfahren zur Herstellung der Verbindungen der Formel XIV, dadurch gekennzeichnet, daß man Verbindungen der Formel X

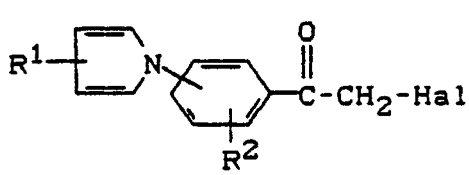

X

in welcher

$R^1$ und $R^2$ die bei den Verbindungen der Formel I angegebenen Bedeutungen haben und Hal für Halogen steht, oxidiert, oder daß man Verbindungen der Formel XXII

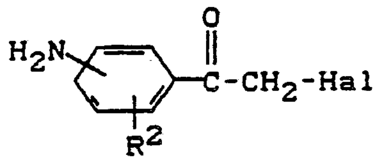

XXII

in welcher
$R^2$ die bei den Verbindungen der Formel I angegebene Bedeutung hat, analog zu den bei Verfahren 2 a-d) genannten Reaktionen umsetzt.

15. Neue Verbindungen der Formel XV

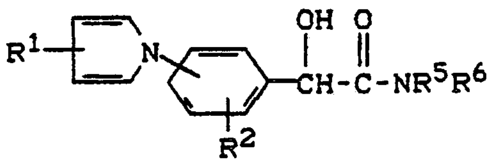

XV

in welcher
$R^1$, $R^2$, $R^5$, $R^6$ die bei den Verbindungen der Formel I angegebenen Bedeutungen besitzen.

16. Verfahren zur Herstellung der Verbindungen der Formel XV in welcher $R^5$ für Wasserstoff steht, dadurch gekennzeichnet, daß man Verbindungen der Formel XVIII

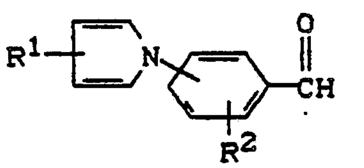

XVIII

in welcher
$R^1$ und $R^2$ die bei den Verbindungen der Formel I angegebenen Bedeutungen besitzen, mit Isonitrilen der Formel XXVI

$CN-R^6$ XXVI

in welcher
$R^6$ die bei den Verbindungen der Formel I angegebene Bedeutung hat, in Gegenwart von Essigsäure umsetzt und die entstandenen O-Acetylverbindungen verseift, oder indem man Verbindungen der Formel XXX

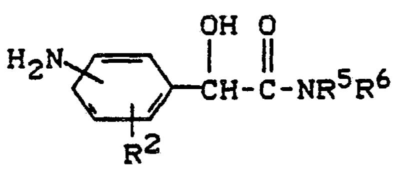

XXX

in welcher
$R^2$, $R^5$, $R^6$ die bei den Verbindungen der Formel I angegebenen Bedeutungen besitzen und $R^5$ und $R^6$ nicht beide gleichzeitig für Wasserstoff stehen dürfen, analog zu den bei Verfahren 2 a-d) genannten Reaktionen umsetzt.

17. Neue Verbindungen der Formel XVIII

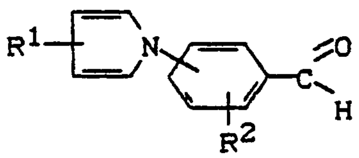

XVIII

in welcher
$R^1$ und $R^2$ die bei den Verbindungen der Formel I angegebenen Bedeutungen haben.

18. Verfahren zur Herstellung der Verbindungen der Formel XVIII, dadurch gekennzeichnet, daß man Verbindungen der Formel XXVII

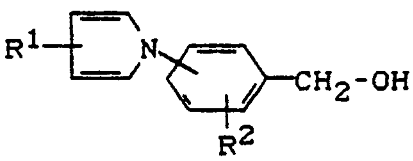

XXVII

in welcher
$R^1$ und $R^2$ die bei den Verbindungen der Formel I angegebenen Bedeutungen haben
oxidiert, oder indem man Säurechloride der Formel XXVIII

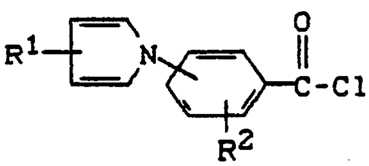

XXVIII

in welcher
$R^1$ und $R^2$ die bei den Verbindungen der Formel I angegebenen Bedeutungen haben
reduziert, oder indem man Verbindungen der Formel XXXI

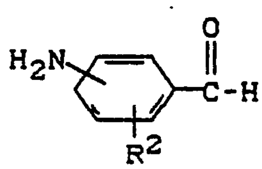

XXXI

in welcher
$R^2$ die bei den Verbindungen der Formel I angegebene Bedeutung hat,
analog zu den bei Verfahren 2 a-d) angegebenen Reaktionen umsetzt.

19. Neue Verbindungen der Formel XIX

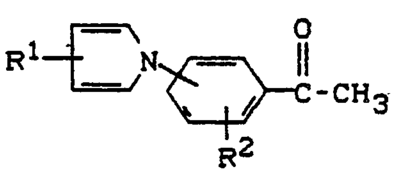

XIX

in welcher
$R^1$ und $R^2$ die bei den Verbindungen der Formel I angegebenen Bedeutungen haben.

20. Verfahren zur Herstellung der Verbindungen der Formel XIX, dadurch gekennzeichnet, daß man Verbindungen der Formel XXIX

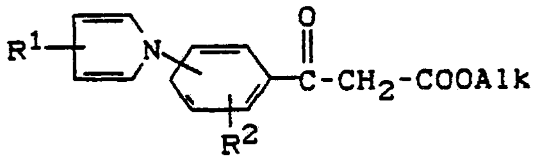

XXIX

in welcher
$R^1$ und $R^2$ die bei den Verbindungen der Formel I angegebenen Bedeutungen besitzen und Alk für $C_{1-4}$-Alkyl steht,
verseift und decarboxyliert, oder indem man Verbindungen der Formel XXXII

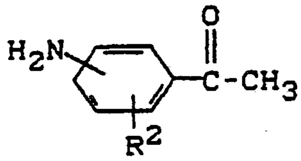

XXXII

in welcher
$R^2$ die bei den Verbindungen der Formel I angegebene Bedeutung hat,
analog zu den bei Verfahren 2 a-d) angegebenen Reaktionen umsetzt.

21. Neue Verbindungen der Formel XXIV

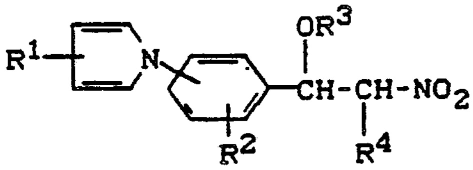

XXIV

in welcher
$R^1$, $R^4$ die bei den Verbindungen der Formel I angegebenen Bedeutungen haben.

22. Verfahren zur Herstellung der Verbindungen der Formel XXIV, dadurch gekennzeichnet, daß man Aldehyde der Formel XVIII

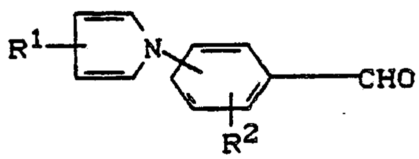

XVIII

in welcher
$R^1$ und $R^2$ die bei den Verbindungen der Formel I angegebenen Bedeutungen haben,
mit Nitromethan umsetzt.

23. Neue Verbindungen der Formel XVII

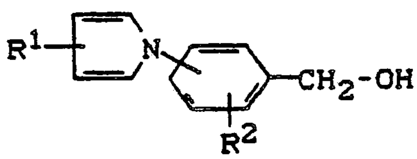

XVII

in welcher
$R^1$ und $R^2$ die bei den Verbindungen der Formel I angegebenen Bedeutungen haben.

24. Verfahren zur Herstellung der Verbindungen der Formel XVII, dadurch gekennzeichnet, daß man Verbindungen der Formel XXXIII

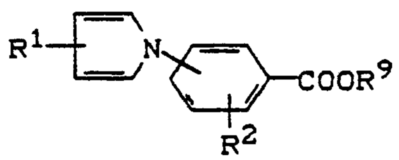

XXXIII

in welcher
$R^1$ und $R^2$ die bei den Verbindungen der Formel I angegebenen Bedeutungen haben und
$R^4$ für Wasserstoff oder $C_{1-4}$-Alkyl steht,
reduziert, oder indem man Verbindungen der Formel XXXIV

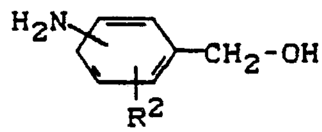

XXXIV

in welcher
$R^2$ die bei den Verbindungen der Formel I angegebene Bedeutung haben
analog zu den bei Verfahren 2 a-d) genannten Reaktionen umsetzt.

25. Neue Verbindungen der Formel XXVIII

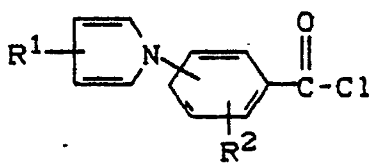

XXVIII

in welcher
$R^1$ und $R^2$ die bei den Verbindungen der Formel I angegebenen Bedeutungen besitzen.

26. Verfahren zur Herstellung der Verbindungen der Formel XXVIII, dadurch gekennzeichnet, daß man Verbindungen der Formel XXXIII

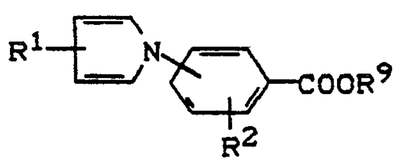

XXXIII

in welcher
$R^1$ und $R^2$ die bei den Verbindungen der Formel I genannten Bedeutungen haben und
$R^9$ für Wasserstoff steht,
mit Halogenierungsmitteln umsetzt, oder indem man Verbindungen der Formel XXXV

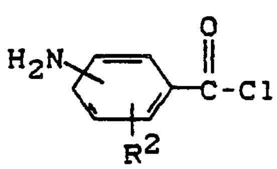

XXXV

in welcher
$R^2$ die bei den Verbindungen der Formel I angegebene Bedeutung hat,

analog zu den bei Verfahren 2 a-d) angegebenen Reaktionen umsetzt.

27. Neue Verbindungen der Formel XXIX

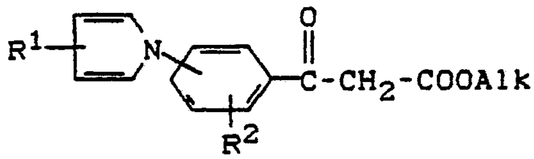

XXIX

in welcher
$R^1$ und $R^2$ die bei den Verbindungen der Formel I genannten Bedeutungen haben und
Alk für $C_{1-4}$-Alkyl steht.

28. Verfahren zur Herstellung der Verbindungen der Formel XXIX, dadurch gekennzeichnet, daß man Verbindungen der Formel XXXIII

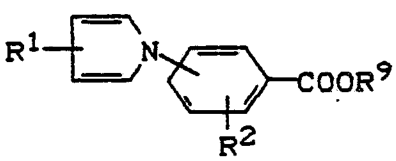

XXXIII

in welcher
$R^1$ und $R^2$ die bei den Verbindungen der Formel I angegebenen Bedeutungen besitzen und
$R^9$ für $C_{1-4}$-Alkyl steht,
mit Essigsäureestern der Formel XXXVI

$CH_3$-COOAlk XXXVI

in welcher
Alk für $C_{1-4}$-Alkyl steht,
umsetzt, oder indem man Verbindungen der Formel XXXVII

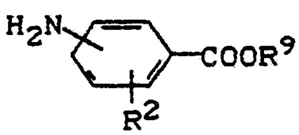

XXXVII

in welcher
$R^2$ die bei den Verbindungen der Formel I angegebenen Reste steht,
analog zu den bei Verfahren 2 a-d) genannten Reaktionen umsetzt.

29. Neue Verbindungen der Formel XXXIII

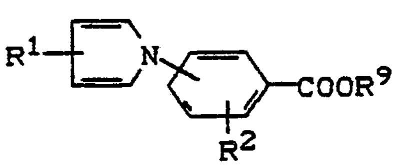

XXXIII

in welcher
$R^1$ und $R^2$ die bei den Verbindungen der Formel I angegebenen Bedeutungen besitzen und
$R^9$ für Waserstoff oder $C_{1-4}$-Alkyl steht.

30. Verfahren zur Herstellung der Verbindungen der Formel XXXIII, dadurch gekennzeichnet, daß man Verbindungen der Formel XXXVII

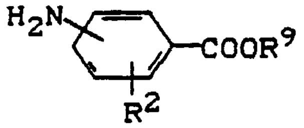

XXXVII

in welcher
$R^2$ die bei den Verbindungen der Formel I angegebene Bedeutung besitzt und
$R^9$ für $C_{1-4}$-Alkyl oder Wasserstoff steht,
analog zu den bei Verfahren 2 a-d) angegebenen Reaktionen umsetzt.

Es wurde ferner gefunden, daß die Verbindungen der Formel I sowie ihre physiologisch verträglichen Salze leistungsfördernde Wirkung bei Tieren, insbesondere eine Wirkung zur Verschiebung des Fleisch/Fett-Verhältnisses zugunsten von Fleisch besitzen. Die Erfindung betrifft die Verwendung der Verbindungen der Formel I in der Tierzucht und Tierernährung.

Die Verbindungen der Formel I können auch in Form ihrer Diastereomere, Racemate oder enantiomeren Formen vorliegen.

Physiologisch verträgliche Salze der Verbindungen der Formel I können mit folgenden Säuren gebildet werden:

Salzsäure, Schwefelsäure, Phosphorsäure, Perchlorsäure, Brom-, Iodwasserstoffsäure, Salpetersäure, Essigsäure, Oxalsäure, Malonsäure, Bernsteinsäure, Ascorbinsäure, Äpfelsäure, Weinsäure, Maleinsäure, Fumarsäure, Methansulfonsäure, Benzoesäure, substituierte Benzoesäuren, Ameisensäure, Toluolsulfonsäure, Benzolsulfonsäure, Phthalsäure, Naphthalinsulfonsäure, Nikotinsäure, Palmitinsäure, Embonsäure.

Bevorzugt sind Verbindungen der Formel I in welcher
$R^1$ für einen oder mehrere gleiche oder verschiedene Reste aus der Gruppe Wasserstoff, Halogen, Cyano, Nitro, Formyl(CHO), Carboxyl(COOH), Carbalkoxyalkyl, Alkoxycarboxyalkyl, $C_{1-4}$-Alkylcarbonyl(Alk-CO-), $C_{1-4}$-Alkoxycarbonyl(Alk-O-CO-), Aminocarbonyl($H_2N$-CO-), $C_{1-4}$-Alkylaminocarbonyl, Di-$C_{1-4}$-alkylaminocarbonyl, $C_{1-4}$-Alkyl, $C_{1-4}$-Alkoxy, $C_{2-6}$-Alkenoxy, $C_{1-4}$-Halogenalkyl, $C_{1-4}$-Halogenalkoxy, $C_{1-4}$-Alkylthio, $C_{1-4}$-Halogenalkylthio, $C_{1-4}$-Hydroxyalkyl, $C_{1-4}$-Cyanoalkyl, $C_{2-8}$-Alkoxyalkyl, $C_{2-8}$-Alkylthioalkyl, $C_{1-4}$-Alkylcarbonyl-$C_{1-4}$-alkoxy steht,
$R^2$ für einen oder mehrere gleiche oder verschiedene Reste aus der Gruppe Wasserstoff, Halogen, Hydroxy, Cyano, Nitro, $C_{1-4}$-Alkyl, $C_{1-4}$-Alkoxy, $C_{1-4}$-Alkylthio, $C_{1-4}$-Halogenalkyl, $C_{1-4}$-Halogenalkoxy, $C_{1-4}$-Halogenalkylthio, $C_{1-4}$-Cyanoalkyl, $C_{2-8}$-Alkoxyalkyl, $C_{1-4}$-Hydroxyalkyl, $C_{1-4}$-Alkoxycarbonyl, Aminocarbonyl, Mono-, Di-$C_{1-4}$-alkylaminocarbonyl steht,
$R^3$ für Wasserstoff, $C_{1-6}$-Alkylcarbonyl, gegebenenfalls substituiertes Benzoyl, $C_{1-6}$-Alkylsulfonyl, gegebenenfalls substituiertes Phenylsulfonyl, Tri-$C_{1-6}$-alkyl-silyl steht,
$R^4$ für Wasserstoff oder Methyl steht,
$R^5$ für Wasserstoff steht,
$R^6$ für geradkettiges, verzweigtes oder cyclisches Alkyl mit bis zu 12 C-Atomen steht, das gegebenenfalls substituiert ist durch Heterocyclyl mit 4 bis 6 Ringatomen und 1 bis 3 Heteroatomen, $C_{1-6}$-Alkoxy, $C_{1-4}$-Alkylthio oder Halogen oder für Cycloalkyl-alkyl mit bis zu 12 C-Atomen, das gegebenenfalls substituiert ist durch Halogen, $C_{1-4}$-Alkoxy oder $C_{1-4}$-Alkylthio.

Besonders bevorzugt sind Verbindungen der Formel I in welcher
$R^1$ für einen oder mehrere gleiche oder verschiedene Reste aus der Gruppe Wasserstoff, $C_{1-4}$-Alkyl, Formyl, $C_{1-4}$-Alkoxycarbonyl, $C_{1-4}$-Alkylcarbonyl steht,
$R^2$ für einen oder mehrere gleiche oder verschiedene Reste aus der Gruppe Wasserstoff, Hydroxy, Cyano, Halogen, insbesondere Chlor, Fluor, Brom, $C_{1-4}$-Alkyl, $C_{1-4}$-Alkoxy, $C_{1-4}$-Alkylthio, $C_{1-4}$-Halogenalkyl insbesondere Trihalogenmethyl, $C_{1-4}$-Halogenalkoxy steht,
$R^3$ für Wasserstoff, $C_{1-6}$-Alkylcarbonyl insbesondere Acetyl, Dimethyl($C_{4-8}$-alkyl)silyl steht,
$R^4$ für Wasserstoff, Methyl steht,
$R^5$ für Wasserstoff steht,
$R^6$ für t-Butyl, i-Propyl, $C_{3-7}$-Cycloalkyl, Dicyclopropylmethyl steht, die gegebenenfalls substituiert sein können durch einen oder mehrere Substituenten aus der Gruppe Halogen, $C_{1-4}$-Alkoxy, $C_{1-4}$-Alkylthio,
ferner steht $R^6$ für den Rest

$$-\overset{CH_3}{\overset{|}{C}}H-A$$

, wobei
A für $C_{3-6}$Cycloalkyl, $C_{4-6}$-Heterocyclyl mit O oder S als Heteratom steht, die gegebenenfalls durch $C_{1-4}$-Alkyl, $C_{1-4}$-Alkoxy oder $C_{1-4}$-Alkylthio substituiert sein können.

Insbesondere seien als Reste $R^6$ genannt t-Butyl, i-Propyl, $C_{1-4}$-Alkoxy-$C_{2-4}$-alkyl, $C_{1-4}$-Alkylthio-

$C_{2-4}$-alkyl, $C_{3-6}$-Cycloalkyl-$C_{2-4}$-alkyl, $C_{2-4}$-Halogenalkyl, Tetrahydropyranyl-$C_{2-4}$-Alkyl, Tetrahydrofuryl-$C_{2-4}$-alkyl, Furyl-$C_{2-4}$-Alkyl, Pyranyl-$C_{2-4}$-alkyl.

Im einzelnen seien folgende Verbindungen der Formel I genannt:

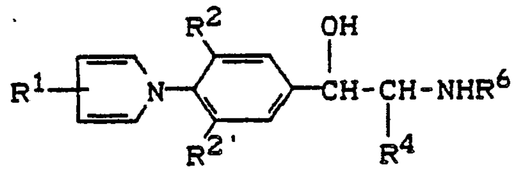

| $R^1$ | $R^2$ | $R^{2'}$ | $R^4$ | $R^6$ |
|---|---|---|---|---|
| $2,4-(CH_3)_2$ | Cl | Cl | H | i-Prop |
| $3-CH_3$ | Cl | Cl | H | $C(CH_3)_3$ |
| $3-C_2H_5$ | Cl | Cl | H | $C(CH_3)_3$ |
| 3-CHO | Cl | Cl | H | $C(CH_3)_3$ |
| 3-COOH | Cl | Cl | H | $C(CH_3)_3$ |
| $3-CH_2COOH$ | Cl | Cl | H | $C(CH_3)_3$ |
| $2-CH_3$ | Cl | Cl | H | $C(CH_3)_3$ |
| H | Cl | Cl | H | C(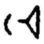 )$_2$ |
| H | Cl | Cl | H | $C(CH_3)$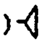 |
| H | Cl | Cl | H | $CH(CH_3)$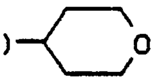 |
| H | Cl | Cl | H | $CH(CH_3)$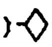 |
| H | Cl | Cl | H | $CH(CH_3)-CH_2OC_2H_5$ |
| H | Cl | Cl | H | $CH(CH_3)$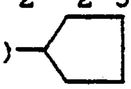 |
| H | Cl | Cl | H | $CH(CH_3)$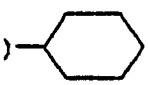 |
| H | Cl | Cl | H | $CH(CH_3)$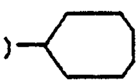 |
| H | Cl | $CH_3$ | H | $C(CH_3)_3$ |
| H | Cl | H | H | $C(CH_3)_3$ |
| H | $CH_3$ | H | H | $C(CH_3)_3$ |
| H | $SCH_3$ | H | H | $C(CH_3)_3$ |
| H | Cl | F | H | $C(CH_3)_3$ |
| H | F | F | H | $C(CH_3)_3$ |
| H | Cl | $CF_3$ | H | $C(CH_3)_3$ |
| H | Cl | $C_2H_5$ | H | $C(CH_3)_3$ |
| H | CN | H | H | $C(CH_3)_3$ |
| H | Cl | Cl | $CH_3$ | $C(CH_3)_3$ |

| $R^1$ | $R^2$ | $R^{2'}$ | $R^4$ | $R^6$ |
|---|---|---|---|---|
| H | Cl | H | $CH_3$ | $C(CH_3)_3$ |
| H | Cl | Cl | $CH_3$ | $CH(CH_3)CH_2OCH_3$ |
| $2.5(CH_3)_2$ | Cl | Cl | $CH_3$ | $CH(CH_3)CH_2OCH_3$ |
| H | Cl | Cl | H | $CH(CH_3)_2CH_2F$ |
| $2.5(CH_3)_2$ | Cl | Cl | H | $CH(CH_3)_2CH_2F$ |
| $2.5(CH_3)_2$ | Cl | Cl | H | $CH(CH_3)$ 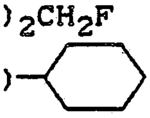 |
| H | Cl | H | H | $CH(CH_3)$ 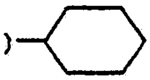 |
| $2.5(CH_3)_2$ | Cl | Cl | H | $CH(CH_3)$ 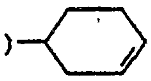 |
| H | Cl | Cl | H | $CH(CH_3)$ 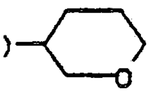 |
| $2.5(CH_3)_2$ | Cl | Cl | H | $CH(CH_3)$ 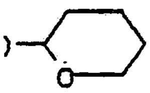 |
| H | Cl | Cl | H | $CH(CH_3)CH_2OCH_3$ |
| $2.5(CH_3)_2$ | Cl | Cl | H | $CH(CH_3)CH_2OCH_3$ |
| $2-CH_3$ | Cl | Cl | H | $CH(CH_3)CH_2OCH_3$ |
| $3-CH_3$ | Cl | Cl | H | $CH(CH_3)CH_2OCH_3$ |
| 3-CHO | Cl | Cl | H | $CH(CH_3)CH_2OCH_3$ |
| 3-COOH | Cl | Cl | H | $CH(CH_3)CH_2OCH_3$ |
| $3-CH_2-COOH$ | Cl | Cl | H | $CH(CH_3)CH_2OCH_3$ |
| H | Cl | $CH_3$ | H | $CH(CH_3)CH_2OCH_3$ |
| H | Cl | H | H | $CH(CH_3)CH_2OCH_3$ |
| H | $CH_3$ | H | H | $CH(CH_3)CH_2OCH_3$ |
| H | $CF_3$ | H | H | $CH(CH_3)CH_2OCH_3$ |
| H | Cl | $CF_3$ | H | $CH(CH_3)CH_2OCH_3$ |
| H | CN | H | H | $CH(CH_3)CH_2OCH_3$ |
| $2.5(CH_3)_2$ | Cl | $CH_3$ | H | $CH(CH_3)CH_2OCH_3$ |
| $2.5(CH_3)_2$ | Cl | H | H | $CH(CH_3)CH_2OCH_3$ |

| $R^1$ | $R^2$ | $R^{2'}$ | $R^4$ | $R^6$ |
|---|---|---|---|---|
| H | Cl | Cl | H | $CH(CH_3)CH_2SCH_3$ |
| H | Cl | Cl | H | $CH(CH_3)$- 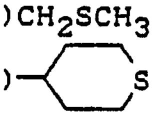 |
| H | Cl | Cl | H | $CH(CH_3)$- 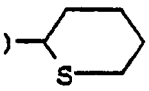 |
| $2.5(CH_3)_2$ | Cl | Cl | H | $CH(CH_3)CH_2SCH_3$ |

$$Ar\text{-}CHOH\text{-}CH_2\text{-}NHR^6$$

| Ar | $R^6$ |
|---|---|
| 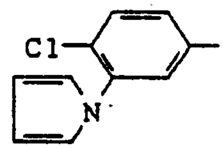 | $C(CH_3)_3$ |
| 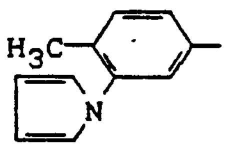 | $C(CH_3)_3$ |
| 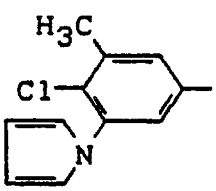 | $C(CH_3)_3$ |
| 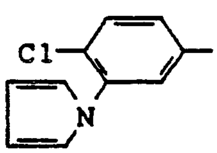 | $CH(CH_3)-CH_2OCH_3$ |

| Ar | $R^6$ |
| --- | --- |
| 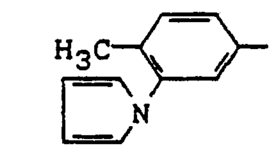 | $CH(CH_3)-CH_2OCH_3$ |
| 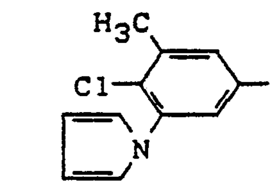 | $CH(CH_3)-CH_2OCH_3$ |
| 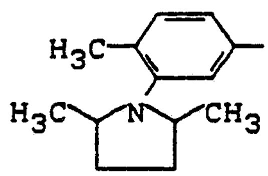 | $CH(CH_3)-CH_2OCH_3$ |
| 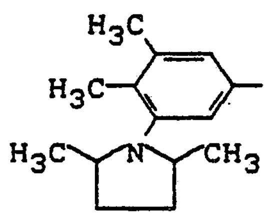 | $CH(CH_3)-CH_2OCH_3$ |

Bevorzugt seien die Salze der Verbindungen der Formel I mit Salzsäure, Schwefelsäure, Phosphorsäure, Oxalsäure, Maleinsäure, Fumarsäure, Malonsäure genannt.

Die Verbindungen der Formel I lassen sich nach den oben angegebenen Verfahren 2a) bis p) herstellen.

Setzt man bei Verfahren 2a) als Verbindung der Formel II 2-(4-Amino-3-chlorphenyl)-N-cyclohexyl-ethanolamin und als Verbindung der Formel III 2,5-Hexandion ein, läßt sich Verfahren a) durch folgendes Reaktionsschema wiedergeben:

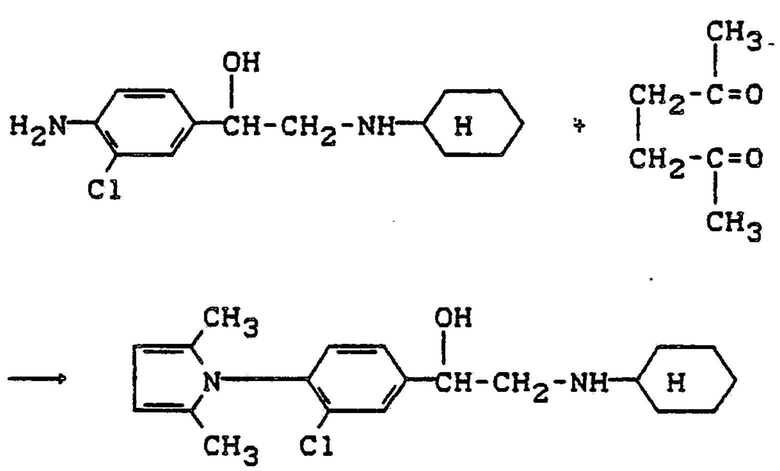

Die Verbindungen der Formel II sind zum Teil bekannt (Arzneim.-Forsch. 34, 1625 (1984); 22, 861/1972). Neue Verbindungen der Formel II sind Gegenstand einer gleichzeitig eingereichten Patentanmeldung der Anmelderin. Sie werden in an sich bekannter Weise hergestellt, z.B. analog zu den bei Verfahren 2e) bis p) genannten Reaktionen. Bevorzugt werden Verbindungen der Formel II eingesetzt, in denen die Reste $R^2$ bis $R^6$ die bei den Verbindungen der Formel I angegebenen bevorzugten und besonders bevorzugten Bedeutungen besitzen. Im einzelnen seien folgende Verbindungen der Formel II genannt:

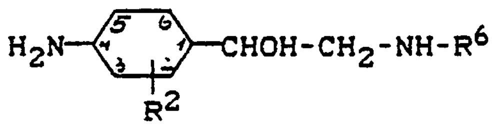

| $R^2$ | $R^6$ |
|---|---|
| 3.5-$Cl_2$ | $C(CH_3)_3$ |
| 3-Cl | $C(CH_3)_3$ |
| 3-$CH_3$ | $C(CH_3)_3$ |
| 3-$CF_3$ | $C(CH_3)_3$ |
| 3-$CF_3$, 5-Cl | $C(CH_3)_3$ |
| 3-CN | $C(CH_3)_3$ |
| 3.5-$Cl_2$ | $CH(CH_3)_2$ |
| 3.5-$Cl_2$ | $CH(CH_3)-CH_2OCH_3$ |
| 3.5-$Cl_3$ | $CH(CH_3)$ 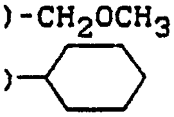 |
| 3-Cl | $CH(CH_3)-CH_2OCH_3$ |
| 3-CN | $CH(CH_3)-CH_2OCH_3$ |
| 3.5-$Cl_2$ | $CH(CH_3)$ 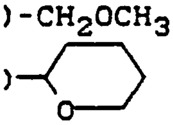 |
| 3.5-$Cl_2$ | $CH(CH_3)$ 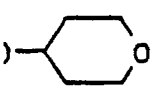 |

Die Verbindungen der Formel III sind bekannt oder lassen sich analog zu bekannten Verfahren herstellen (vgl. Houben-Weyl Methoden der Organischen Chemie Band 7/2b S. 1876 ff; Band 6/3 Seite 707 ff; Band 7/1 S. 255 ff). Wie die Verbindungen der Formel III lassen sich auch deren Monoacetale, Diacetale oder cyclischen Acetale der Formeln IIIa, b, c einsetzen.

Bevorzugt werden Verbindungen der Formel III eingesetzt, in der $R^1$ die bei den Verbindungen der Formel I angegebenen bevorzugten und besonders bevorzugten Bedeutungen besitzt. Im einzelnen seien folgende Verbindungen der Formel III genannt:

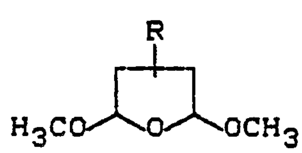

R = H, 2.5-$(CH_3)_2$, 2-$CH_3$, 3-$CH_3$, 3-CHO, 3-COOH, 3-$CH_2$-COOH, 3-$CH_2$-$COOCH_3$, 3-$CH_2$-$COOC_2H_5$, 3.4-

$(COOC_2H_5)_2$

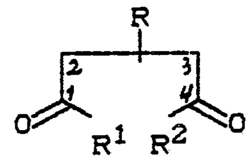

| R | $R^1$ | $R^2$ |
|---|---|---|
| H | $CH_3$ | $CH_3$ |
| H | $CH_3$ | $C_2H_5$ |
| $2-CH_3$ | $CH_3$ | $CH_3$ |
| H | H | $CH_3$ |
| H | H | H |

Bevorzugt seien die Methyl-oder Ethylacetale genannt wie z.B.:

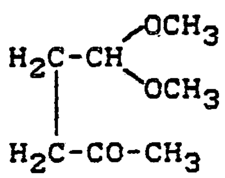 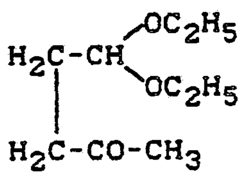

Bevorzugt seien die cyclischen Acetale genannt wie z.B.:

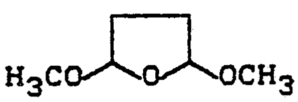 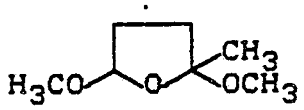 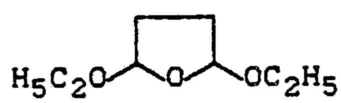

Die Umsetzung wird analog zu den für die Knorr-Paal Synthese bekannten Bedingungen durchgeführt (Synthesis 1976 S. 295; The Chemistry of Pyrroles, S. 77 ff).

Sie wird bevorzugt in folgenden Lösungsmittel durchgeführt: Kohlenwasserstoffe wie Toluol, Säuren wie Eisessig, Nitrile wie Acetonitril, Ester wie Essigsäureethylester.

Sie wird vorteilhaft in Gegenwart saurer Katalysatoren durchgeführt. Als solche seien genannt: Essigsäure, p-Toluolsulfonsäure, Chlorwasserstoffsäure, Methansulfonsäure.

Sie wird bei Temperaturen von 0 - 150° C, bevorzugt zwischen 20 und 100° C, vorteilhafterweise am Wasserabscheider durchgeführt.

Die Ausgangsprodukte werden in etwa äquimolarem Verhältnis zueinander eingesetzt.

Die Aufarabeitung erfolgt indem man das Reaktionsgemisch auf Wasser gießt, alkalisch stellt, mit Lösemitteln wie Essigester, Ether, Methylenchlorid extrahiert, die organische Phase eindampft, chromatographiert oder umlöst oder abdestilliert.

Setzt man cyclische Acetale der Formel IIIc als Ausgangsprodukte ein, wird vorteilhaft in Gegenwart saurer Katalysatoren gearbeitet. Als solche seien genannt: Essigsäure, p-Toluolsulfonsäure, Methansulfonsäure.

Die Umsetzung von Verbindungen der Formel II mit Dihalogenketonen der Formel IV in Verfahren 2b) läßt sich im Fall von 2-[4-Amino-5-trifluormethylthiophenyl]-N-cyclobutylaminoethanol und Dibromallen durch folgendes Formelschema darstellen:

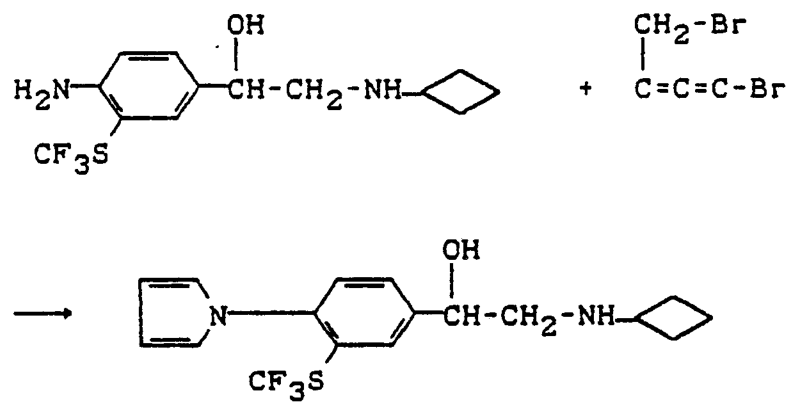

Das Verfahren wird in an sich bekannter Weise durchgeführt. Verbindungen der Formel IV sind bekannt oder lassen sich analog zu bekannten Verfahren herstellen.

Die Umsetzung von Verbindungen der Formel II mit Epoxybutanen der Formel V im Verfahren 2c) läßt sich im Fall von 2-(4-Amino-5-cyanophenyl)-N-1-ethyltetrahydrofurylamino-propanol(2) und 3,4-Epoxybutyraldehyddiethylacetal durch folgendes Formelschema wiedergegeben:

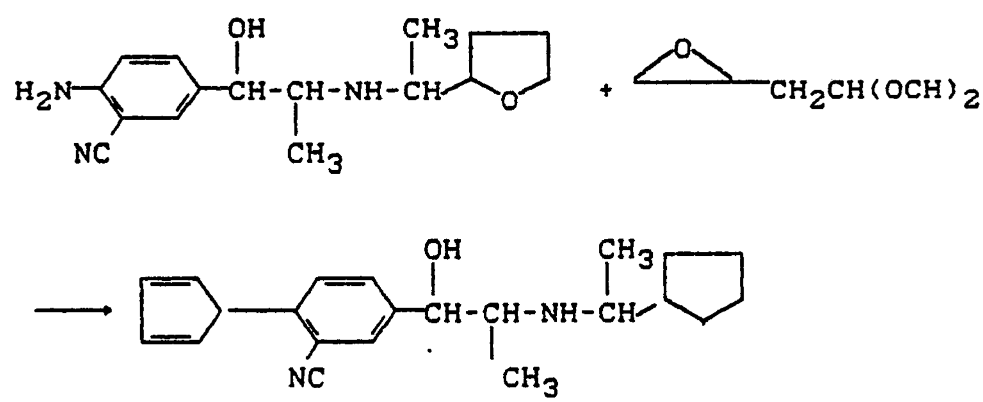

Das Verfahren wird in an sich bekannter Weise durchgeführt. Die Epoxybutane der Formel V sind bekannt oder lassen sich analog zu bekannten Verfahren herstellen.

Die Umsetzung von Verbindungen der Formel II mit Halogencrotonaldehyd im Verfahren 2d) läßt sich im Fall von 4-Amino-3-fluor-5-ethyl-phenyl-t-butylaminoethanol und 2-Chlorcrotonaldehyd durch folgendes Reaktionsschema darstellen:

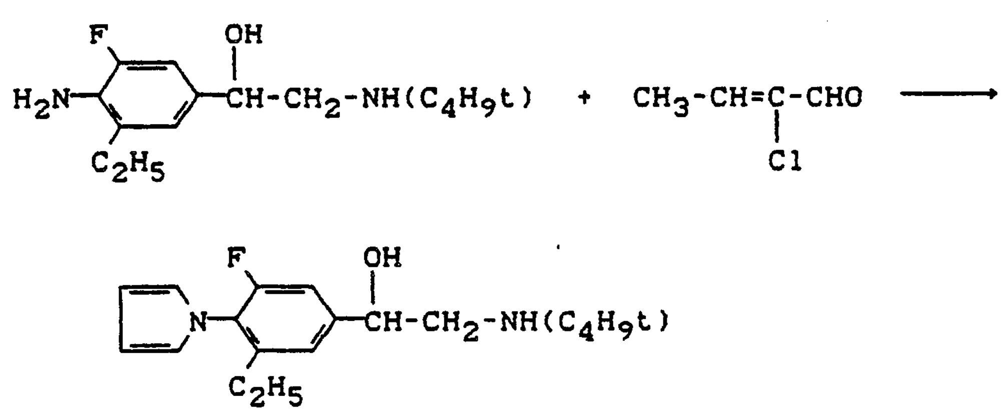

Das Verfahren wird in an sich bekannter Weise durchgeführt. Die Halogencrotonaldehyde der Formel VI sind bekannt oder lassen sich analog zu bekannten Verfahren herstellen.

Setzt man bei Verfahren 2e) als Verbindung der Formel VII 2-(3-Pyrrolo-5-methoxyphenyl-)-2-oxocyclohexylethylamin ein, läßt sich Verfahren 2e) durch folgendes Formelschema wiedergeben:

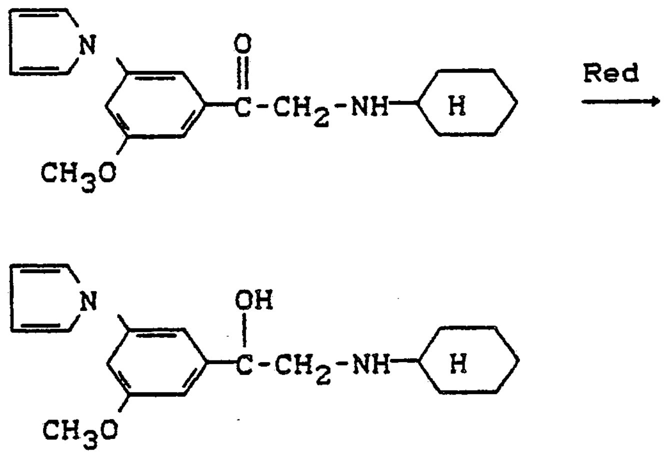


Die Verbindungen der Formel VII sind neu. Ihre Herstellung erfolgt nach dem weiter unten (4) beschriebenen Verfahren.

Die Substituenten $R^1$, $R^2$, $R^4$, $R^5$, $R^6$ in Formel VII besitzen bevorzugt die bei den Verbindungen der Formel I genannten bevorzugten und besonders bevorzugten Bedeutungen. Im einzelnen seien folgende Verbindungen der Formel VII genannt:

| $R^1$ | $R^2$ | $R^4$ | $R^5$ | $R^6$ |
|---|---|---|---|---|
| H | 3.5-$Cl_2$ | H | H | $CMe_3$ |
| H | 3.5-$Cl_2$ | H | H | $CH(CH_3)CH_2OCH_3$ |
| H | 3-Cl | H | H | $CH(CH_3)CH_2OCH_3$ |
| H | 3.5-$Cl_2$ | H | H | $CH(CH_3)$ 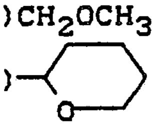 |

Als Reduktionsmittel zur Durchführung des Verfahrens seien folgende Reduktionsmittel genannt:

$H_2$/Katalysator, als Katalysator seien beispielsweise genannt: $PtO_2$, Pd-Aktivkohle:

komplexe Metallhydride wie z.B. $LiAlH_4$, $NaBH_4$, $NaBH_3CN$.

Besonders bevorzugt werden folgende Reduktionsmittel eingesetzt:

$NaBH_4$ und $NaBH_3CN$

Die Reaktion wird bei Temperaturen von -20°C bis +100°C bevorzugt zwischen 0 und 50°C durchgeführt.

Es wird bevorzugt bei Normaldruck gearbeitet.

Als Verdünnungsmittel dienen alle inerten organischen Lösungsmittel. Hierzu gehören insbesondere aliphatische und aromatische Kohlenwasserstoffe wie Pentan, Hexan, Cyclohexan, Petrolether, Ligroin, Benzol, Toluol; chlorierte Kohlenwasserstoffe wie Methylenchlorid, Ethylenchlorid, Chloroform; Ether wie Diethylether und Glykoldimethylether; Nitrile wie Acetonitril, Propionitril und Benzonitril; Alkohole wie Methanol, Ethanol, n-und i-Propanol. Bevorzugt sind Alkohole.

Setzt man bei Verfahren 2f) als Epoxid der Formel VII 3-Methyl-4-pyrrolophenyl-epoxid und als Amin der Formel IX t-Butylamin ein, läßt sich Verfahren 2f) durch folgendes Reaktionsschema wiedergeben:

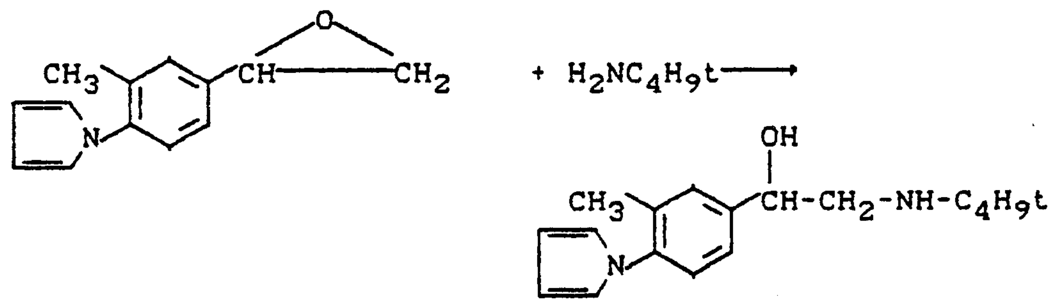

Epoxide der Formel VII sind neu. Ihre Herstellung erfolgt nach dem unter (6) beschriebenen Verfahren. Bevorzugt werden Epoxide der Formel VIII eingesetzt in welche $R^1$, $R^2$ und $R^4$ die bei den Verbindungen der Formel I genannten bevorzugten und besonders bevorzugten Bedeutungen besitzen.

Im einzelnen seien die folgenden Epoxide genannt:

4-Pyrrolo-3-chlorphenyl-epoxid,
4-Pyrrolo-3-cyanophenyl-epoxid,
3,5-Dichlor-4-pyrrolophenyl-epoxid,
3-Chlor-4-pyrrolo-5-cyanophenyl-epoxid,
3-Cyano-4-pyrrolo-5-chlorphenyl-epoxid,
3-Cyano-4-pyrrolo-phenyl-epoxid,
3-Chlor-4-pyrrolo-5-trifluormethylphenyl-epoxid,
3-Brom-4-pyrrolo-5-cyanophenyl-epoxid.

Bevorzugt werden Amine der Formel IX eingesetzt, in welchen $R^5$ und $R^6$ die bei den Verbindungen der Formel I genannten bevorzugten und besonders bevorzugten Bedeutungen besitzen.

Als Amine der Formel IX seien genannt z.B. tert.-Butylamin, Isopropylamin, 2-Methoxymethyl-ethylamin, 2-Tetrahydropyranylethylamin, 2-Cyclohexylethylamin, 2-Cyclobutylethylamin, 2-Cyclopropyl-ethylamin.

Verfahren 2f) wird durchgeführt indem man etwa äquimolare Mengen des Epoxids der Formel VIII und des Amins der Formel IX in einem Verdünnungsmittel umsetzt.

Im allgemeinen wird ein Überschuß an Amin (1-3 molar, bevorzugt 1-1,5 molar) bezogen auf das Epoxid der Formel VIII verwendet.

Die Reaktion wird bei Temperaturen von +20 bis +150°C durchgeführt.

Es wird bevorzugt bei Normaldruck gearbeitet.

Als Verdünnungsmittel dienen alle inerten organischen Lösungsmittel. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl-und Dibutylether, Glykoldimethylether und Diglykoldimethylether, Tetrahydrofuran und Dioxan, ferner Nitrile wie Acetonitrile und Benzonitril, Amide wie Dimethylformamid, Alkohole wie Methanol, Ethanol, n-und i-Propanol.

Bevorzugt sind Alkohole.

Setzt man bei Verfahren 2g) als 2-Halogenketon der Formel X 3-Chlor-4-(2-Ethoxycarbonylpyrrol)-chloracetophenon und als Amin der Formel IX 1-Cyclohexylethylamin ein, läßt sich Verfahren 2g) durch folgendes Formelschema wiedergeben:

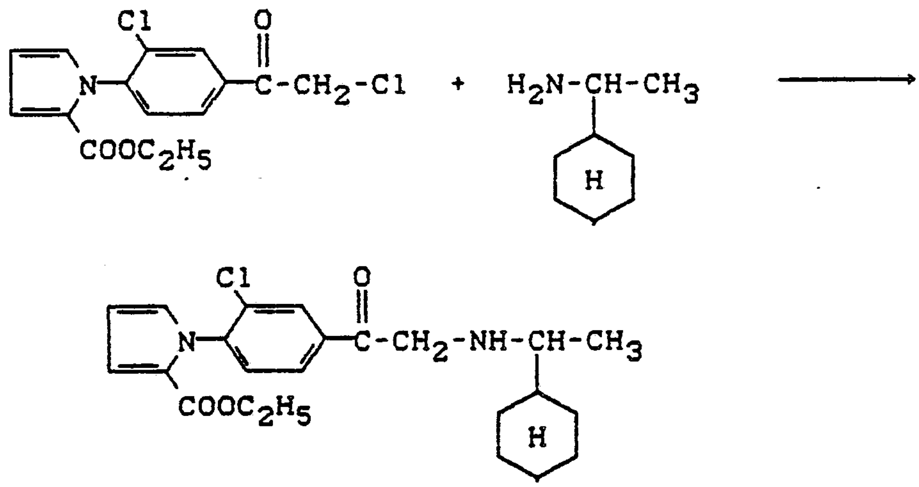

Die 2-Halogenketone der Formel X sind neu. Ihre Herstellung erfolgt nach dem unter (8) beschriebenen Verfahren. Bevorzugt werden Verbindungender Formel X eingesetzt, in welcher $R^1$, $R^2$ die bei den Verbindungen der Formel I genannten bevorzugten und besonders bevorzugten Bedeutungen besitzen und Halogen für Chlor oder Brom steht.

Bevorzugt werden die bei Verfahren 2f) genannten Amine eingesetzt.

Im einzelnen seien folgende Verbindungen der Formel X genannt:

(2-Pyrrolo-3-chlorphenyl-5-chlormethylketon,
(2-Pyrrolo-3-cyanophenyl-5-chlormethylketon,
(2,4-Dichlor-3-pyrrolophenyl-6-brommethylketon,
(2-Cyano-3-pyrrolophenyl-6-brommethylketon,
(3-Pyrrolo-4-cyanophenyl-6-brommethylketon,
(2-Pyrrolo-3-cyanophenyl-5-brommethylketon,
(2-Cyano-3-pyrrolo-4-chlorphenyl-brommethylketon,
(2-Cyano-3-pyrrolo-4-chlorphenyl-6-chlormethylketon,
(2-Chlor-3-pyrrolo-4-trifluormethylphenyl-6-brommethylketon,
(2-Trifluormethyl-3-pyrrolo-4-cyanophenyl-6-brommethylketon,
(2-Fluor-3-pyrrolo-4-cyanophenyl-6-chlormethylketon.

Verfahren 2g) wird durchgeführt indem man etwa äquimolare Mengen des 2-Halogenketons der Formel X und des Amins der Formel IX in einem Verdünnungsmittel umsetzt. Im allgemeinen wird ein Überschuß an Amin (1-3 molar, bevorzugt 1-1,5 molar) bezogen auf das 2-Halogenketon der Formel X verwendet.

Die Reaktion wird bei Temperaturen von 20 bis 150°C, bevorzugt 50 bis 120°C, durchgeführt.

Als bevorzugte Verdünnungsmittel seinen die bei Verfahren 2f) genannten Verdünnungsmittel genannt.

Setzt man bei Verfahren 2h) als beta-Halogenmethylverbindung der Formel XI 3-(1-Hydroxy-2-chlorethyl)-methoxyphenylpyridin und als Amin der Formel IX t-Butylamin ein, läßt sich Verfahren 2h) durch folgendes Formelschema wiedergeben:

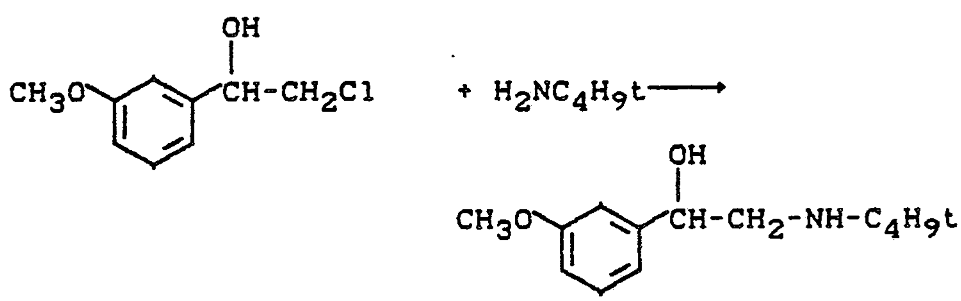

Beta-Halogenmethylverbindungen der Formel XI sind neu. Ihre Herstellung erfolgt nach dem unter (10) beschriebenen Verfahren. Bevorzugt sind Verbindungen der Formel IX, in welcher $R^1$, $R^2$, $R^4$ die bei den Verbindungen der Formel I genannten bevorzugten Bedeutungen besitzen und Hal für Chlor oder Brom

steht.

Im einzelnen seien folgende Verbindungen der Formel XI genannt:
1-(2-Pyrrolo-3-chlor-phenyl)-2-chlorethanol,
1-(2-Pyrrolo-3-cyano-phenyl)-2-chlorethanol,
1-(2,4-Dichlor-3-pyrrolophenyl)-2-chlorethanol,
1-(2-Chlor-3-pyrrolo-4-cyano-phenyl)-2-chlorethanol,
1-(2-Cyano-3-pyrrolo-4-chlor-phenyl)-2-bromethanol,
1-(2-Cyano-3-pyrrolophenyl)-2-chlorethanol,
1-(3-Pyrrolo-4-cyano-phenyl)-2-bromethanol,
1-(2-Chlor-3-pyrrolo-4-trifluormethyl-phenyl)-2-chlorethanol,
1-(2-Cyano-3-pyrrolo-4-fluor-phenyl)-2-bromethanol.

Verfahren 2h) wird durchgeführt indem man die beta-Halogenmethylverbindung der Formel XI mit überschüssigem Amin der Formel IX gegebenenfalls in Gegenwart eines Verdünnungsmittels umsetzt.

Die Reaktion wird bei Temperaturen von +20 bis +150°C durchgeführt.

Es wird bei Normaldruck oder unter erhöhtem Druck gearbeitet.

Als Verdünnungsmittel dienen alle inerten organischen Lösungsmittel. Hierzu gehören insbesondere aliphatische und aromatische, gegebenenfalls halogenierte Kohlenwasserstoffe, wie Pentan, Hexan, Cyclohexan, Benzol, Toluol, Methylenchlorid, Chloroform, ferner Ether wie Diethylether, Tetrahydrofuran und Dioxan, weiterhin Nitrile wie Acetonitril und Benzonitril, ferner Amide wie Dimethylformamid, ferner Alkohole wie Methanol, Ethanol, n-und i-Propanol.

Bevorzugt eingesetzt werden Alkohole.

Setzt man bei Verfahren 2i) als Verbindung der Formel XII 3-Fluor-4-pyrrolo-(1-hydroxy-2-aminoethyl)-phenyl und als Verbindung der Formel XIII Acetophenon ein, läßt sich Verfahren 2i) durch folgendes Formelschema wiedergeben:

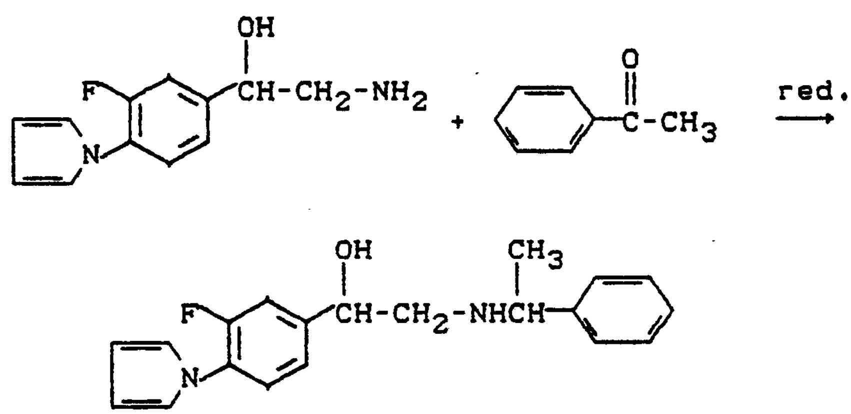


Verbindungen der Formel XII sind neu. Ihre Herstellung erfolgt nach dem unter (12) beschriebenen Verfahren. Bevorzugt werden Verbindungen der Formel XII eingesetzt, in welcher $R^1$, $R^2$, $R^3$, $R^4$ die bei den Verbindungen der Formel I genannten bevorzugten und besonders bevorzugten Bedeutungen besitzen.

Im einzelnen seien folgende Verbindungen der Formel XII genannt:

1-(2-Pyrrolo-3-chlor-phenyl)-2-aminoethanol,
1-(2-Pyrrolo-3-cyano-phenyl)-2-aminoethanol,
1-(2,4-Dichlor-3-pyrrolophenyl)-2-aminoethanol,
1-(2-Chlor-3-pyrrolo-4-cyano-phenyl)-2-aminoethanol,
1-(2-Cyano-3-pyrrolophenyl)-2-aminoethanol,
1-(2-Chlor-3-pyrrolo-4-trifluormethyl-phenyl)-2-aminoethanol.

Verbindungen der Formel XIII sind bekannt oder lassen sich analog zu bekannten Verfahren herstellen.

$R^{13}$ steht bevorzugt für $C_{1-4}$-Alkyl, insbesondere Methyl, $C_{3-6}$-Cycloalkyl, insbesondere Cyclopropyl,

$R^{14}$ steht bevorzugt für $C_{1-4}$-Alkyl, des gegebenenfalls substituiert ist durch Halogen, insbesondere Fluor, $C_{1-4}$-Alkoxy, insbesondere Methoxy, $C_{1-4}$-Alkylthio, insbesondere Methylthio,

ferner für $C_{3-6}$-Cycloalkyl, das gegebenenfalls substituiert ist durch $C_{1-4}$-Alkyl, insbesondere Methyl, $C_{1-4}$-Alkoxy, insbesondere Methoxy,

ferner für Heterocyclyl mit 4 - 6 Ringatomen und O als Heteroatom, das gegebenenfalls substituiert ist durch $C_{1-4}$-Alkyl, insbesondere Methyl, wie z.B. Tetrahydrofuranyl, Furanyl, Pyranyl, Tetrahydropyranyl,

ferner stehen $R^{13}$ und $R^{14}$ gemeinsam mit dem C-Atom an das sie gebunden sind, für einen aliphatischen Ring mit 3 - 7 Ringatomen wie Cyclopropyl, Cyclopentyl, Cyclohexyl, Cyclohexenyl.

Im einzelnen seien folgende Verbindungen der Formel XIII genannt: Aceton, Methylethylketon, Methylisopropylketon, Diethylketon, Ethylpropylketon, Methylcyclohexylketon, Cyclohexanon.

Verfahren 2i) wird durchgeführt indem man etwa äquimolare Mengen der Verbindungen der Formeln XII und XIII in einem Verdünnungsmittel vorlegt und die Mischung reduziert.

Die Reaktion wird bei Temperaturen von 0°C bis 150°C durchgeführt.

Es wird bevorzugt bei Normaldruck gearbeitet.

Als Verdünnungsmittel dienen alle inerten organischen Lösungsmittel. Hierzu gehören aliphatische und aromatische, gegebenenfalls halogenierte Kohlenwasserstoffe wie Pentan, Hexan, Cyclohexan, Benzol, Toluol, Methylenchlorid, Ethylenchlorid, Chloroform, Chlorbenzol, ferner Ether wie Diethylether, Tetrahydrofuran, Dioxan, weiterhin Nitrile wie Acetonitril, Benzonitril, Amide wie Dimethylformamid, Alkohole wie Methanol, Ethanol.

Als Reduktionsmittel dienen: $H_2$/Katalysator, als Katalysator sei beispielsweise $PtO_2$ genannt; komplexe Metallhydride wie z.B. $LiAlH_4$, $NaBH_4$, $NaBH_3CN$; Raney-Nickel.

Setzt man bei Verfahren 2j) als Verbindung der Formel XIV 3-Methyl-4-pyrrolophenylglyoxal und als Amin der Formel III t-Butylamin ein, läßt sich Verfahren 2j) durch folgendes Formelschema wiedergeben.

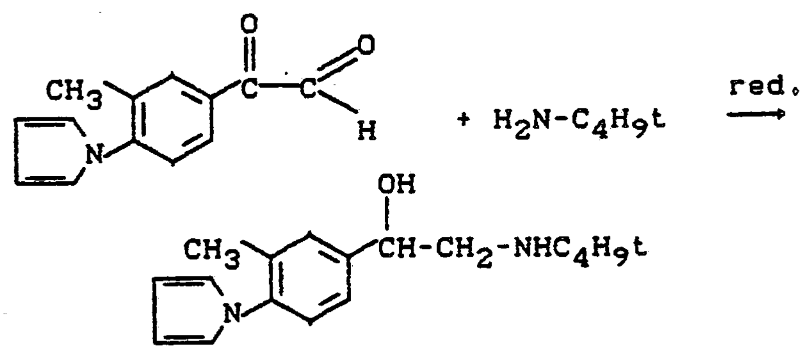


Die Verbindungen der Formel XIV sind neu. Ihre Herstellung erfolgt nach dem unter (14) beschriebenen Verfahren.

Die Substituenten $R^1$, $R^2$ in Formel XIV besitzen bevorzugt die weiter oben bei den Verbindungen der Formel I angegebenen bevorzugten Bedeutungen. Im einzelnen seien folgende Verbindungen der Formel XIV genannt:

4-Pyrrolo-3-chlor-phenylglyoxal,
4-Pyrrolo-3-cyano-phenylglyoxal,
3,5-Dichlor-4-pyrrolophenylglyoxal,
3-Cyano-4-pyrrolophenylglyoxal,
3-Chlor-4-pyrrolo-5-trifluormethyl-phenylglyoxal,

Verfahren 2j) wird durchgeführt, indem man zur Verbindung der Formel XIV in einem Verdünnungsmittel etwa die äquivalente Menge des Amins der Formel IX zusetzt und anschließend reduziert.

Die Reaktion wird bei Temperaturen durchgeführt von 0°C bis 100°C.

Es wird bevorzugt bei Normaldruck gearbeitet.

Als Verdünnungsmittel dienen alle inerten organischen Lösungsmittel. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl-und Dibutylether, Glykoldimethylether und Di glykoldimethylether, Tetrahydrofuran und Dioxan, außerdem Ester wie Essigsäuremethylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Tetramethylensulfon und Hexamethylphosphorsäuretriamid, weiterhin Alkohole wie Methanol, Ethanol, n-und i-Propanol.

Als Reduktionsmittel dienen $H_2$/Katalysator (als Katalysator seien $PtO_2$ und Pd-Kohle genannt), ferner komplexe Metallhydride wie $LiAlH_4$ und $NaBH_4$.

Setzt man bei Verfahren 2k) als Verbindung der Formel XV (5-Chlor-4-pyrrolophenyl)-hydroxyessigsäureisopropylamid ein, läßt sich Verfahren 2k) durch folgendes Reaktionsschema wiederge-

ben:

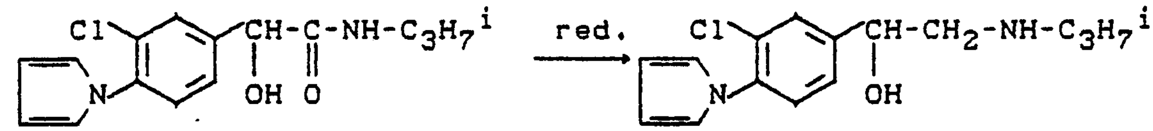


Verbindungen der Formel XV sind neu. Ihre Herstellung erfolgt nach dem unter (16) beschriebenen Verfahren. Bevorzugt werden Verbindungen der Formel XV eingesetzt, in welcher $R^1$, $R^2$, $R^5$, $R^6$ die bei den Verbindungen der Formel I genannten bevorzugten und besonders bevorzugten Bedeutungen besitzen

Im einzelnen seien die folgenden Verbindungen der Formel XV genannt:

(3-Chlor-4-pyrrolophenyl)hydroxyessigsäureisopropylamid,
(3-Cyano-4-pyrrolophenyl)hydroxyessigsäureisopropylamid,
(3-Chlor-4-pyrrolopphenyl)hydroxyessigsäure-tert.-butylamid,
(3-Chlor-4-pyrrolo-5-amino-cyano-phenyl)hydroxyessigsäure-isopropylamid,
(3-Cyano-4-pyrrolo-5-chlor-phenyl)hydroxyessigsäure-tert.-butylamid
(3-Cyano-4-pyrrolo-5-chlor-phenyl)hydroxyessigsäureisopropylamid,
(3,5-Dichlor-4-pyrrolophenyl)hydroxyessigsäure-tert.-butylamid,
(3,5-Dichlor-4-pyrrolophenyl)hydroxyessigsäureisopropylamid,
(3,5-Dichlor-4-pyrrolophenyl)hydroxyessigsäure-tert.-butylamid.

Verfahren 2k) wird durchgeführt, indem man die Verbindung der Formel XV in einem Verdünnungsmittel mit überschüssigem Reduktionsmittel umsetzt.

Die Reaktion wird bei Temperaturen von 0°C bis +150°C durchgeführt.

Es wird bevorzugt bei Normaldruck gearbeitet.

Als Verdünnungsmittel dienen alle inerten organischen Lösungsmittel. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl-und Dibutylether, Glykoldimethylether und Diglykoldimethylether, Tetrahydrofuran und Dioxan.

Als Reduktionsmittel dienen komplexe Metallhydride wie $LiAlH_4$, Borane wie Diboran.

Setzt man bei Verfahren 2l) als Verbindung der Formel I 4-(2-Ethylpyrrolo)-3,5-difluorphenylethanol-dicyclopropylmethylamin und als Acylierungsmittel Benzoylchlorid ein, so läßt sich Verfahren 2l) durch folgendes Reaktionsschema wiedergeben:

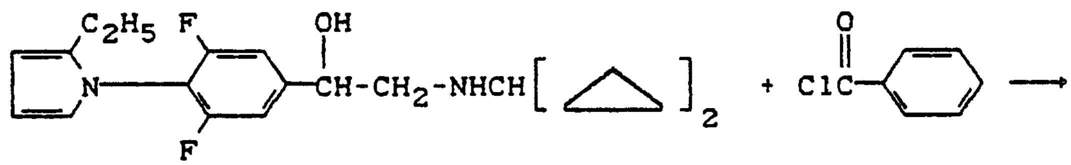

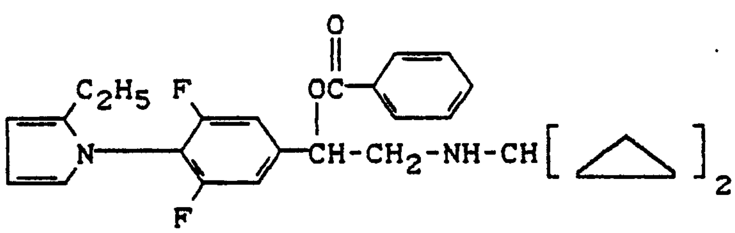

Die Acylierungsreaktion wird analog zu bekannten Acylierungsreaktionen durchgeführt.

Als Acylierungsmittel seien bevorzugt genannt: Acetylchlorid, Acetanhydrid, Propionsäureanhydrid, Methoxyacetylchlorid.

Verbindungen der Formel I und die Acylierungsmittel werden bevorzugt in Gegenwart von Verdünnungsmitteln sowie in Gegenwart von Säurebindemitteln umgesetzt.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan,

Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl-und Dibutylether, Glykoldimethylether und Diglykoldimethylether, Tetrahydrofuran und Dioxan, weiterhin Ketone, wie Aceton, Methylethyl-, Methylisopropyl-und Methylisobutylketon, außerdem Ester, wie Essigsäure-methylester und -ethylester, ferner Nitrile, wie z.B. Acetonitril und Propionitril, Benzontril, Glutarsäuredinitril, darüber hinaus Amide, wie z.B. Dimethylformamid, Dimethylacetamid und N-methylpyrrolidon, sowie Dimethylsulfoxid, Tetramethylensulfon und Hexamethylphosphorsäuretriamid.

Als Säurebindemittel seien genannt: Alkali-, Erdalkalialkoholate und tertiäre Amine. Besonders bevorzugt seien folgende Basen genannt: Triethylamin, Pyridin, Picoline, Trimethylamin, N-Methyl-morpholin, N-Ethyl-pyrrolidon, Diazabicyclo(4,3,0)undecen (DBU), 1,4-Diaza-bicyclo-2,2,2-octan (DABCO), Diazabicyclo-(3,2,0)nonen (DBN).

Die Reaktion wird durchgeführt bei 0 - 150°C, bevorzugt bei 20 - 100°C, vorzugsweise unter Normaldruck.

Bevorzugt wird unter Schutzgasatmosphäre gearbeitet.

Die Verbindungen der Formel I und die Acylierungsmittel werden bevorzugt in äquimolarem Verhältnis zueinander eingesetzt. Ein Überschuß der einen oder anderen Verbindung bringt keinen wesentlichen Vorteil. Die Säurebindemittel werden bevorzugt äquimolar oder in einem Überschuß bis zu 10 molar, bezogen auf die Verbindungen der Formel I eingesetzt. Werden tertiäre Amine als Säurebindemittel eingesetzt, können diese auch als Reaktionsmedium dienen.

Die Aufarbeitung erfolgt in an sich bekannter Weise, inde das ausgeschiedene Salz abfiltriert wird und die organische Phase eingeengt wird oder indem das Reaktionsgemisch in Wasser eingegossen wird und die organische Phase nach Abtrennung eingeengt wird.

Setzt man bei Verfahren 2m) als Verbindung der Formel I 3-Cyano-4-(2-methoxypyrrolo)-phenyl-2-cyclopentylamino-propanol und als Silylierungsmittel der Formel XVI Dimethyl-isovaleryl-silylchlorid ein, läßt sich Verfahren 2m) durch folgendes Reaktionsschema wiedergeben:

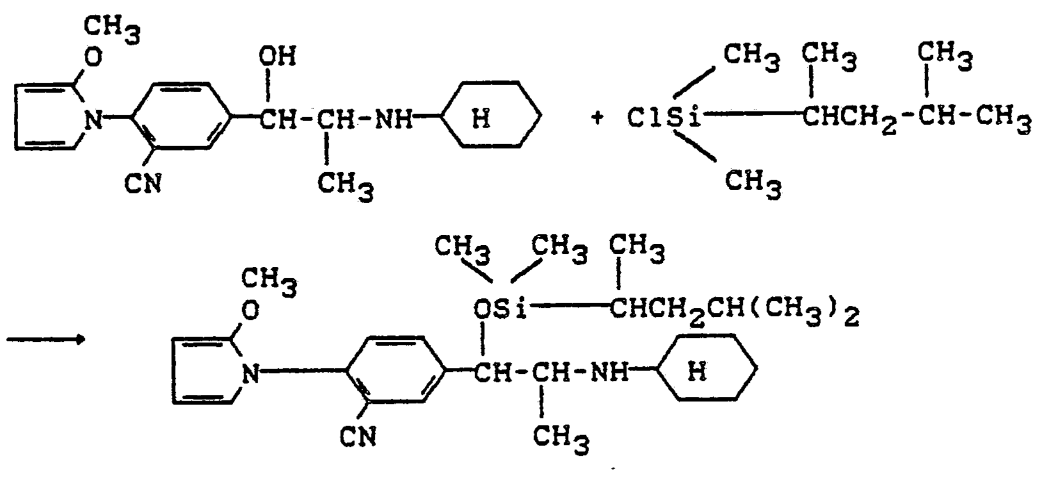

Die Verbindungen der Formel I können nach einem der obengenannten Verfahren 2a-e) hergestellt werden. Die Verbindungen der Formel XVI sind bekannt.

Das Verfahren wird analog zu bekannten Methoden der organischen Chemie durchgeführt.

Das Verfahren kann in Gegenwart von Verdünnungsmitteln durchgeführt werden. Als Verdünnungsmittel kommen alle inerten organischen Lösungsmittel infrage. Hierzu gehören insbesondere aliphatische und aromatische, gegebenenfalls halogenierte Kohlenwasserstoffe, wie Pentan,. Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether, wie Diethyl-und Dibutylether, Glykoldimethylether und Diglykoldimethylether, Tetrahydrofuran und Dioxan, weiterhin Ketone, wie Aceton, Methylethyl-, Methylisopropyl-und Methylisobutylketon, außerdem Ester, wie Essigsäure-methylester und -ethylester, ferner Nitrile, wie z.B. Acetonitril und Propionnitril, Benzonitril, Glutarsäuredinitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid.

Das Verfahren kann in Gegenwart von Katalysatoren durchgeführt werden. Als Katalysatoren können bevorzugt verwendet werden: Imidazol, Triazol oder Diisopropylethylamin.

Die Reaktionstemperatur wird zwischen etwa 0°C und 130°C, vorzugsweise zwischen etwa 20°C und

60°C, gehalten. Das Verfahren wird vorzugsweise bei Normaldruck durchgeführt.

Die Ausgangsverbindungen werden im allgemeinen in etwa äquimolarem Verhältnis eingesetzt.

Setzt man bei Verfahren 2n) als Verbindung der Formel I 4-Pyrrolo-3-trifluormethyl-5-chlorphenyl-ethanol-methoxyisopropylamin und Phosgen ein, so läßt sich Verfahren 2n) durch folgendes Formelschema wiedergeben:

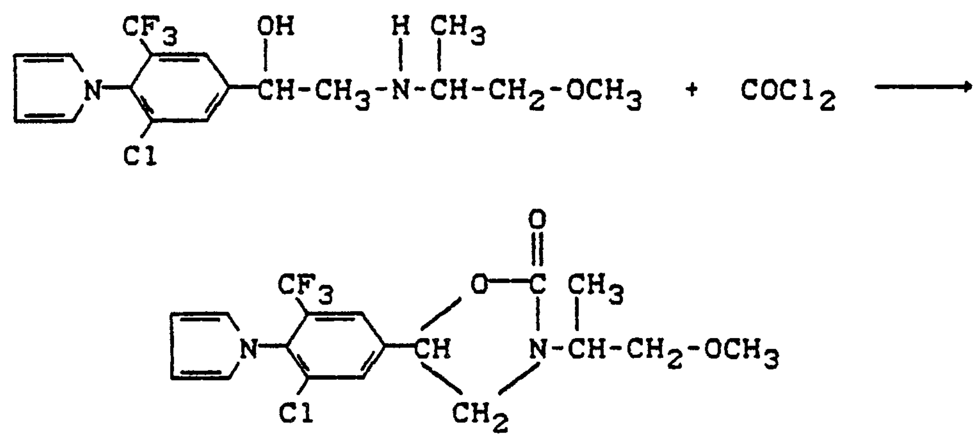

Das Verfahren wird durchgeführt indem man Phenylethanolamine der Formel I mit Phosgen oder Phosgen-abspaltenden Mitteln gegebenenfalls in Anwesenheit von Basen wie z.B. tertiären Aminen, bevorzugt Triethylamin oder Imidazol, zwischen 0 - 100°C in inerten Verdünnungsmitteln wie z.B. gegebenenfalls halogenierten Kohlenwasserstoffen, bevorzugt Methylenchlorid oder Toluol, umsetzt.

Setzt man bei Verfahren 2o) als Verbindung der Formel I 4-(3,4-Dimethylpyrrolo)-3-methoxyphenyl-ethanol-monofluortertiärbutylamin und als Aldehyd der Formel XVII Acetaldehyd ein, so läßt sich Verfahren 2o) durch folgendes Formelschema wiedergeben:

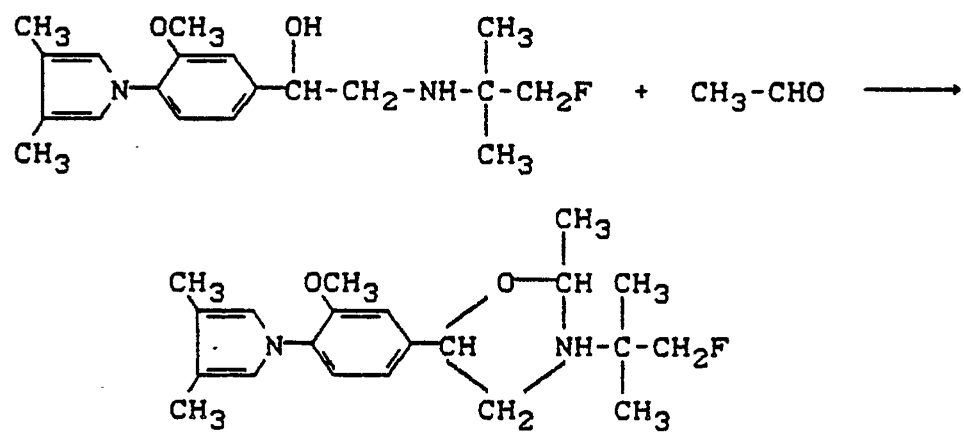

Das Verfahren wird durchgeführt, indem man Phenylethanolamine der Formel I mit Aldehyden oder Ketonen, bevorzugt Formaldehyd, Aceton, gegebenenfalls in Gegenwart von sauren Katalysatoren wie z.B. p-Toluolsulfonsäure oder in Gegenwart von Molekularsieben gegebenenfalls in inerten Verdünnungsmitteln wie z.B. gegebenenfalls halogensubstituierten Kohlenwasserstoffen zwischen 0 - 100°C umsetzt.

Verfahren 2p) wird unter den für die Alkylierung von Aminen mit Alkylhalogeniden üblichen Bedingungen durchgeführt. Bevorzugt wird in inerten Verdünnungsmiteln wie Kohlenwasserstoffen, z.B. Toluol, Xylol, Ethern wie Dioxan, Tetrahydrofuran, Ketonen wie Aceton, bei Temperaturen von 20-180°C, bevorzugt zwischen 60 und 150°C, gearbeitet. Gegebenenfalls wird unter Zusatz von Basen wie anorganischen Basen, z.B. Alkali-und Erdalkalihydroxiden, -carbonaten oder -hydrogencarbonaten, organischen Basen wie tertiären Aminen, z.B. Triethylamin, gearbeitet.

Die Verbindungen der Formel XXXVIII werden meist äquimolar zu den Verbindungen der Formel XII eingesetzt. Es kann vorteilhaft sein, einen 1-bis 3-fachen Überschuß an Verbindungen der Formel XXXVIII einzusetzen.

Die Verbindungen der Formel XXXVIII sind bekannt oder können nach an sich bekannten Verfahren

hergestellt werden. $R^{13}$ und $R^{14}$ besitzen bevorzugt die weiter oben bei den Verbindungen der Formel XIII angegebenen bevorzugten und besonders bevorzugten Bedeutungen.

Als Verbindungen der Formel XII werden bevorzugt die weiter oben bei Verfahren 2i) bevorzugt eingesetzten Verbindungen der Formel XII verwendet.

Wie bereits erwähnt lassen sich die neuen Verbindungen der Formel VII nach dem unter 4 angegebenen Verfahren herstellen.

Verfahren 4 wird durchgeführt wie bei Verfahren 2f) in der ersten Stufe beschrieben. Es werden bevorzugt die bei Verfahren 2g) genannten Verbindungen der Formel X und Amine der Formel IX eingesetzt.

Wie bereits erwähnt, lassen sich die neuen Verbindungen der Formel VIII nach den unter (6) angegebenen Verfahren herstellen.

Verfahren 6 wird durchgeführt, indem man eine Verbindung der Formel XI in einem Verdünnungsmittel mit der 2-5 molaren, bevorzugt 2-4 molaren Menge einer Base umsetzt. Setzt man als Verbindung der Formel XI 1-(3-Chlor-4-pyrrolophenyl)-2-bromethanol und als Base NaOH ein, läßt sich die Reaktion durch folgendes Schema darstellen:

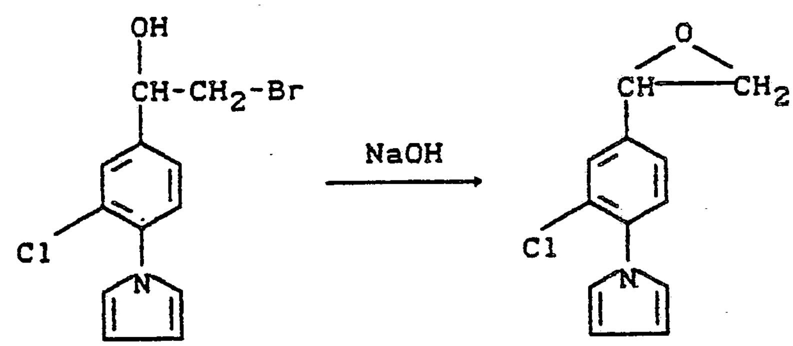


Verbindungen der Formel XI sind neu. Sie werden nach dem unter (10) beschriebenen Verfahren hergestellt.

Bevorzugt werden Verbindungen der Formel XI eingesetzt, in welcher $R^1$, $R^2$, $R^4$ die bei den Verbindungen der Formel I angegebenen bevorzugten Bedeutungen besitzen und Hal für Chlor oder Brom steht.

Im einzelnen seien folgende Verbindungen der Formel XI genannt:

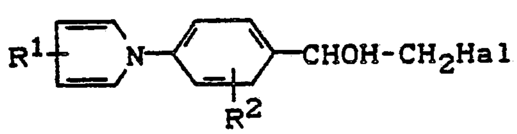

| $R^1$ | $R^2$ | Hal |
|---|---|---|
| H | $3,5\text{-}Cl_2$ | Cl |
| H | $3,5\text{-}Cl_2$ | Br |
| $2,5(CH_3)_2$ | $3,5\text{-}Cl_2$ | Cl |
| H | 3-Cl | Cl |
| $2,5(CH_3)_2$ | 3-Cl | Br |

Als Basen seien genannt: Alkali-und Erdalkalihydroxide wie Natriumhydroxid, Kaliumhydroxid; -carbonate und -hydrogencarbonate wie Natriumcarbonat, Natriumhydrogencarbonat, Bariumcarbonat; -alkoholate wie Natriummethylat und Natriumethylat.

Als Verdünnungsmittel seien genannt Alkohole, wie Methanol, Ethanol, Wasser, sowie Mischungen von Alkoholen mit Wasser.

Die Reaktion wird bei Temperaturen von 0°C bis +100°C durchgeführt, es wird bevorzugt unter Normaldruck gearbeitet.

Setzt man bei Verfahren 6 als Verbindung der Formel XVIII 3-Methyl-4-pyrrolobenzaldehyd und als methylengruppenübertragendes Reagenz Trimethylsulfoniumiodid sowie als Base Natriumhydrid ein, läßt sich die Umsetzung durch folgendes Schema darstellen:

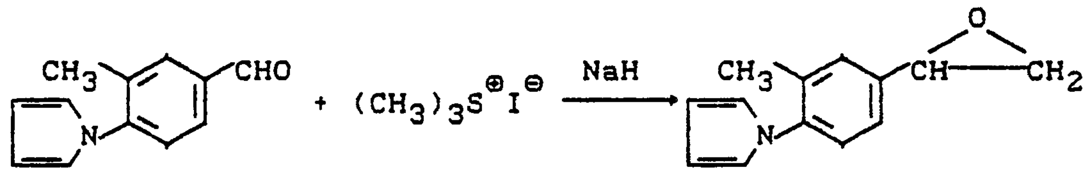


Verbindungen der Formel XVIII sind neu. Sie werden nach dem unter (18) genannten Verfahren hergestellt. Bevorzugt werden Verbindungen der Formel XVIII eingesetzt, in welcher $R^1$ und $R^2$ die bei den Verbindungen der Formel I genannten bevorzugten Bedeutungen besitzten.

Im einzelnen seien folgende Verbindungen der Formel XVIII genannt:

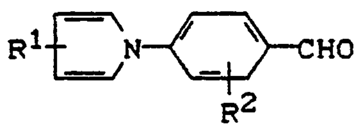

| $R^1$ | $R^2$ | $R^1$ | $R^2$ |
|---|---|---|---|
| H | 3.5-$Cl_2$ | 2.5($CH_3$)$_2$ | 3.5-$Cl_2$ |
| H | 3-Cl | 2.5($CH_3$)$_2$ | 3-Cl |
| H | 3-$CF_3$ 5-Cl | 2.5($CH_3$)$_2$ | 3-$CF_3$ 5-Cl |
| H | 3-$CH_3$ | 2.5($CH_3$)$_2$ | 3-CN |
| H | 3-CN | | |

Als methylengruppenübertragende Reagentien seien genannt:

Trimethylsulfoniumhalogenide wie Trimethylsulfoniumchlorid, -bromid und -iodid,

Trimethylsulfoxoniumhalogenide wie Trimethylsulfoxoniumchlorid, -bromid und -iodid.

Als Basen werden verwendet: Alkali-und Erdalkalihydride wie Natriumhydrid, Alkali-und Erdalkalialkoholate wie Kalium-tert.-butylat.

Das Verfahren wird durchgeführt, indem man 1,1-Äquivalente der Base z.B. in Dimethylsulfoxid vorlegt, dann das methylengruppenübertragende Agens (1,1 Äquivalente) zufügt und zuletzt 1 Äquivalent der Verbindung der Formel XVIII zugibt.

Die Reaktion wird bei Temperaturen von 0°C bis 100°C, bevorzugt bei 50-70°C durchgeführt, es wird bevorzugt bei Normaldruck gearbeitet.

Als Verdünnungsmittel werden Dimethylsulfoxid oder Mischungen von Dimethylsulfoxid mit inerten organischen Lösungsmitteln eingesetzt.

Als inerte organische Lösungsmittel seien genannt: Ether wie Diethylether, Tetrahydrofuran, Dioxan.

Die neuen Halogenmethylketone der Formel X lassen sich nach den unter (8) genannten Verfahren herstellen.

Die Verbindungen der Formel XIX sind neu. Sie lassen sich nach dem unter (20) beschriebenen Verfahren herstellen. Bevorzugt werden Verbindungen der Formel XIX eingesetzt, in welcher $R^1$ und $R^2$ die bei den Verbindungen der Formel I angegebenen bevorzugten und besonders bevorzugten Bedeutungen besitzen.

Im einzelnen seien folgende Verbindungen der Formel XIX genannt:

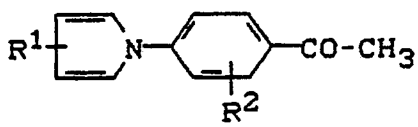

| $R^1$ | $R^2$ | $R^1$ | $R^2$ |
|---|---|---|---|
| H | 3.5-$Cl_2$ | 2.5($CH_3$)$_2$ | 3.5-$Cl_2$ |
| H | 3-Cl | 2.5($CH_3$)$_2$ | 3-Cl |
| H | 3-$CF_3$ 5-Cl | 2.5($CH_3$)$_2$ | 3-$CF_3$, 5-Cl |
| H | 3-$CH_3$ | 2.5($CH_3$)$_2$ | 3-$CH_3$ |
| H | 3-CN | 2.5($CH_3$)$_2$ | 3-CN |
| H | 3-$CF_3$ | 2.5($CH_3$)$_2$ | 3-$CF_3$ |

Setzt man bei Verfahren 8 als Verbindung der Formel XIX 4-Fluor-5-pyrroloacetophenon und als Halogen Hal Brom ein, läßt sich die Reaktion durch folgendes Schema darstellen:

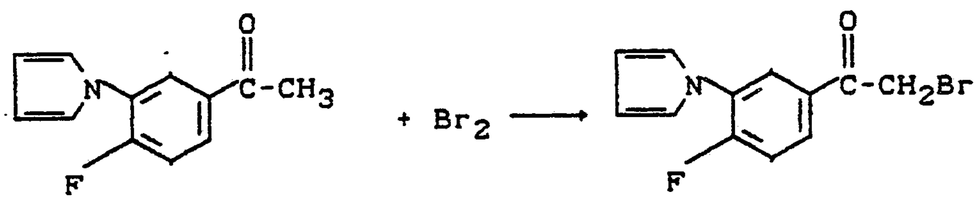


Verfahren 8 wird durchgeführt, indem man zu Verbindung XIX in einem Verdünnungsmittel die äquivalente Menge Halogen, eventuell in einem Verdünnungsmittel gelöst, zugibt.

Die Reaktion wird bei +20°C bis +150°C durchgeführt, bevorzugt bei der Siedetemperatur des verwendeten Verdünnungsmittels.

Es wird bevorzugt bei Normaldruck gearbeitet.

Als Verdünnungsmittel seien genannt: aliphatische, gegebenenfalls halogenierte Kohlenwasserstoffe wie Pentan, Hexan, Heptan, Cyclohexan, Methylenchlorid, Ethylenchlorid, Chloroform, Tetrachlorkohlenstoff, Alkohole wie Methanol, Ethanol, Ester wie Essigsäureethylester und Mischungen aus diesen Verdünnungsmitteln.

Setzt man bei Verfahren 8 als Verbindung der Formel XIX 3-Chlor-4-pyrroloacetophenon und als Kupferhalogenid Kupfer(II)bromid ein, läßt sich die Reaktion durch folgendes Schema darstellen:

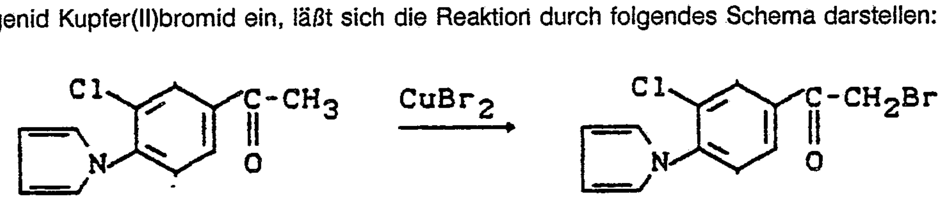


Das Verfahren wird durchgeführt, indem man äquivalente Mengen der Verbindung der Formel XIX und Kupferhalogenid im Verdünnungsmittel 1-24 h, bevorzugt 6-12 h unter Rückfluß erhitzt.

Reaktionsparameter und Verdünnungsmittel sind wie oben beschrieben.

Wie bereits erwähnt, lassen sich die neuen Verbindungen der Formel XI nach dem unter (10) angegebenen Verfahren herstellen.

Setzt man bei Verfahren 10 als Verbindung der Formel X 3-Cyano-4-pyrrolochloracetophenon ein, läßt sich Verfahren 10 durch das folgende Reaktionsschema darstellen:

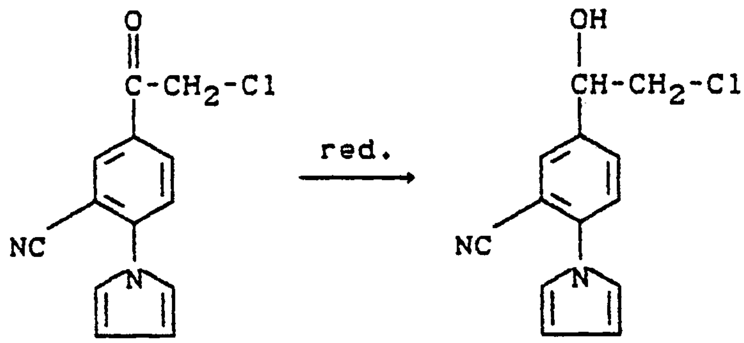


Die Substituenten R¹, R² in den Verbindungen der Formel X haben die bei den Verbindungen der Formel I angegebenen bevorzugten Bedeutungen. Hal steht bevorzugt für Chlor oder Brom.

Als Reduktionsmittel zur Durchführung des Verfahrens seien genannt:

$H_2$/Katalysator (als Katalysatoren seien genannt: $PtO_2$, Pd/Kohle), komplexe Metallhydride, wie z.B. $LiAlH_4$, $NaBH_4$, $NaBH_3CN$. Bevorzugt werden $NaBH_4$ und $NaBH_3CN$ eingesetzt.

Verfahren 10 wird durchgeführt, indem man die Verbindung X in einem Verdünnungsmittel mit dem Reduktionsmittel umsetzt.

Die Reaktion wird bei Temperaturen von -20°C bis +100°C durchgeführt.

Es wird bevorzugt bei Normaldruck gearbeitet.

Als Verdünnungsmittel dienen alle inerten organischen Lösungsmittel. Hierzu zählen insbesondere gegebenenfalls halogenierte aliphatische und aromatische Kohlenwasserstoffe wie Pentan, Hexan, Cyclohexan, Benzol, Toluol, Methylenchlorid, Chloroform, Chlorbenzol; Ether wie Diethylether, Tetrahydrofuran; Nitrile wie Acetonitril, Benzonitril; Alkohole wie Methanol, Ethanol, n-und i-Propanol. Bevorzugt werden Alkohole eingesetzt.

Wie bereits erwähnt, lassen sich die Verbindungen der Formel XII nach dem unter 12 angegebenen Verfahren herstellen.

Setzt man bei Verfahren 12 als Nitroverbindung der Formel XXIV 1-(3-Brom-4-pyrrolophenyl)-2-nitroethanol ein, läßt sich die Reaktion durch das folgende Schema darstellen:

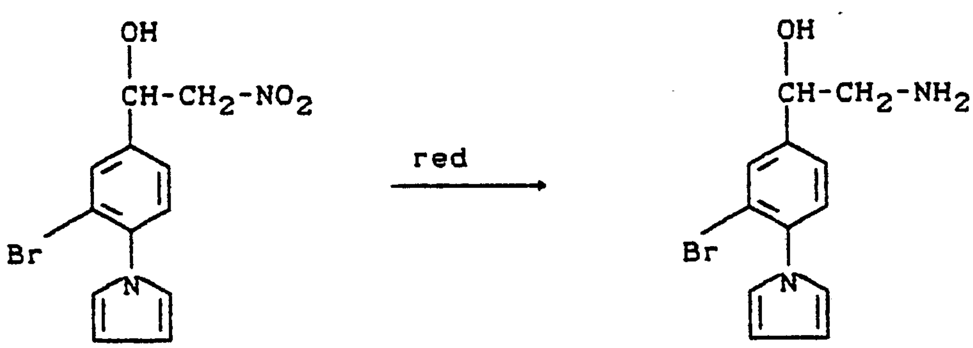


Die Verbindungen der Formel XXIV sind neu. Sie lassen sich nach dem unter (22) angegebenen Verfahren herstellen. Die Substituenten R¹, R², R³ und R⁴ in Formel XXIV besitzen bevorzugt die weiter oben bei den Verbindungen der Formel I angegebenen bevorzugten Bedeutungen

Im einzelnen seien folgende Verbindungen der Formel XXIV genannt:

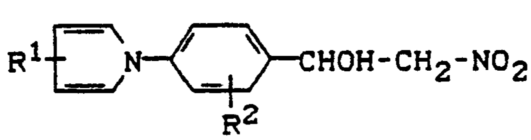

| $R^1$ | $R^2$ | $R^1$ | $R^2$ |
|---|---|---|---|
| H | 3.5-$Cl_2$ | 2.5($CH_3$)$_2$ | 3.5-$Cl_2$ |
| H | 3-Cl | 2.5($CH_3$)$_2$ | 3-Cl |
| H | 3-$CF_3$ | 2.5($CH_3$)$_2$ | 3-$CF_3$ |
| H | 3-CN | 2.5($CH_3$)$_2$ | 3-$CH_3$ |
| H | 3-$CF_3$, 5-Cl | 2.5($CH_3$)$_2$ | 3-CN |
| | | 2.5($CH_3$)$_2$ | 3-$CF_3$, 5-Cl |

Als Reduktionsmittel für das Verfahren dient Wasserstoff/ Katalysator. Als Katalysatoren seien beispielhaft genannt: Raney-Nickel, $PtO_2$, Pd/Kohle.

Das Verfahren wird durchgeführt, indem man Verbindung XXI in einem Verdünnungsmittel unter Zusatz einer Säure katalytisch hydriert.

Die Reaktion wird bei Temperaturen von +20°C bis +150°C durchgeführt.

Es wird unter Normaldruck oder erhöhtem Druck gearbeitet.

Als Verdünnungsmittel dienen alle inerten organischen Lösungsmittel. Hierzu gehören insbesondere aliphatische und aromatische Kohlenwasserstoffe wie Pentan, Hexan, Cyclohexan, Benzol, Toluol; Ether wie Diethylether, Tetrahydrofuran; Alkohole wie Methanol, Ethanol.

Als Säuren werden verwendet: anorganische Säuren wie Kohlensäure; Halogenwasserstoffsäuren wie Chlorwasserstoff; Schwefelsäure, organische Säuren wie Essigsäure, Propionsäure.

Wie bereits erwähnt lassen sich die neuen Verbindungen der Formel XIV nach dem unter (14) genannten Verfahren herstellen.

Setzt man bei dem Verfahren als Halogenmethylketon der Formel X 3-Cyano-4-tetramethylpyrrolobromacetophenon ein, läßt sich das Verfahren durch das folgende Schema darstellen:

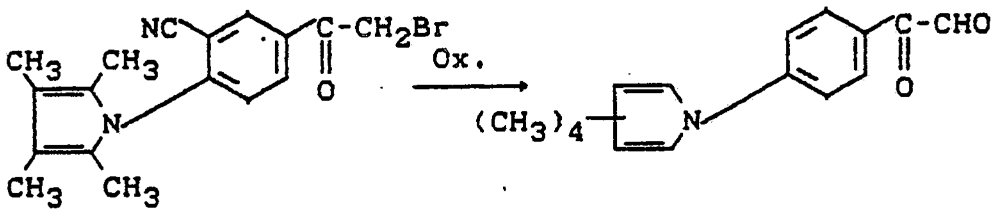


Als Halogenmethylketone der Formel X werden bevorzugt die weiter oben genannten Verbindungen eingesetzt.

Das Verfahren wird durchgeführt, indem man die Verbindung X, gegebenenfalls in Gegenwart eines Verdünnungsmittels, oxidiert.

Die Reaktion wird bei Temperaturen von +20°C bis +100°C durchgeführt.

Es wird bevorzugt bei Normaldruck gearbeitet.

Als Oxidationsmittel wird bevorzugt Dimethylsulfoxid verwendet (N. Kornblum et. al., JACS 79, 6562 (1957).

Wie die Umsetzung in Gegenwart eines Verdünnungsmittels durchgeführt, können alle inerten organischen Lösungsmittel verwendet werden. Hierzu gehören insbesondere aliphatische und aromatische, gegebenenfalls halogenierte Kohlenwasserstoffe wie Pentan, Hexan, Cyclohexan, Benzol, Toluol, Methylenchlorid, Chloroform, Chlorbenzol; Ether wie Diethylether, Tetrahydrofuran; Nitrile wie Acetonitril, Benzonitril. Bevorzugt wird in Dimethylsulfoxid ohne ein weiteres Lösungsmittel gearbeitet.

Wie bereits erwähnt lassen sich die neuen Verbindungen der Formel XV nach dem unter 16 genannten Verfahren herstellen.

Setzt man bei dem Verfahren 16 als Aldehyd der Formel XVIII 3-Fluor-4-pyrrolobenzaldehyd und als Isonitril der Formel XXVI Isopropylisonitril ein, so läßt sich das Verfahren durch das folgende Schema darstellen:

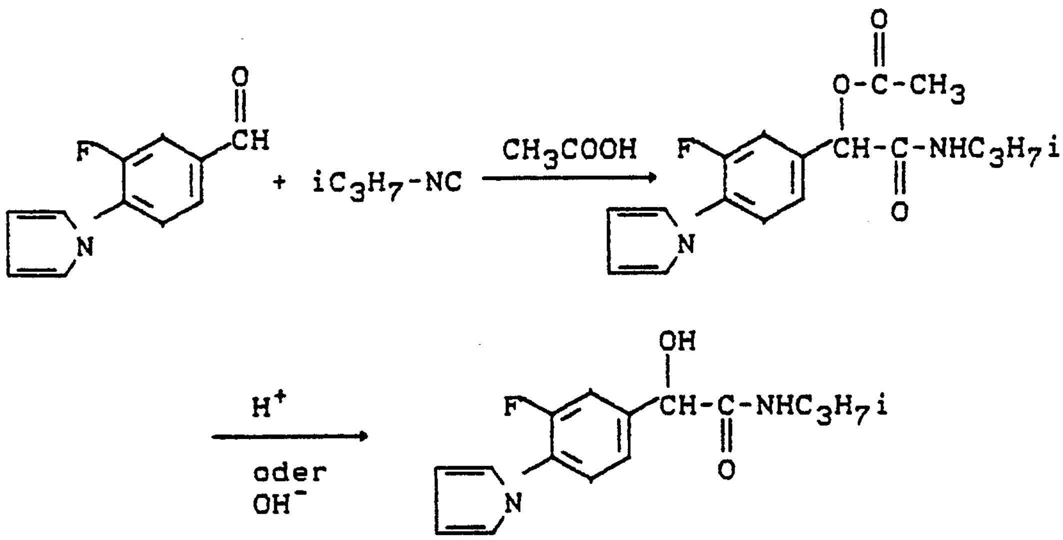


Bevorzugt werden Aldehyde der Formel XVIII eingesetzt, in der $R^1$ und $R^2$ die bei den Verbindungen der Formel I angegebenen bevorzugten Bedeutungen besitzen. Die Aldehyde der Formel XVIII sind neu. Sie lassen sich nach dem unter (18) angegebenen Verfahren herstellen.

Isonitrile der Formel XXVI sind bekannt oder lassen sich analog zu bekannten Verfahren herstellen. Der Substituent $R^6$ besitzt bevorzugt die weiter oben bei den Verbindungen der Formel I angegebenen bevorzugten Bedeutungen. Im einzelnen seien folgende Verbindungen der Formel XXVI genannt: Methylisonitril, Ethylisonitril, n-Propylisonitril, Isopropylisonitril, n-Butylisonitril, sec.-Butylisonitril, Isobutylisonitril, tert.-Butylisonitril.

Das Verfahren wird durchgeführt, indem man die Verbindung XVIII mit der doppelt molaren Menge von Isonitril der Formel XXVI und Essigsäure in einem Verdünnungsmittel zusammengibt.

Die Reaktion wird bei Temperaturen von +20°C bis +150°C durchgeführt.

Es wird bevorzugt bei Normaldruck gearbeitet.

Als Verdünnungsmittel dienen alle inerten organischen Lösungsmittel. Hierzu gehören insbesondere gegebenenfalls halogenierte aliphatische und aromatische Kohlenwasserstoffe wie Pentan, Hexan, Cyclohexan, Benzol, Toluol, Methylenchlorid, Chloroform, Chlorbenzol; Ether wie Diethylether, Tetrahydrofuran; Nitrile wie Acetonitril und Benzonitril.

Zur Abspaltung der Acetylgruppe werden anorganische Säuren verwendet. Hierzu zählen Halogenwasserstoffsäuren wie Salzsäure; Schwefelsäure, Phosphorsäure. Die Abspaltung kann auch in Gegenwart anorganischer Basen wie Alkali-oder Erdalkalihydroxide, -carbonate oder -hydrogencarbonate erfolgen.

Das Verfahren wird durchgeführt, indem man die acetylierte Verbindung direkt oder nach vorheriger Isolierung in einem Verdünnungsmittel als Lösungsvermittler mit überschüssiger wäßriger Lösung der anorganischen Säure oder Base behandelt.

Die Reaktion wird bei Temperaturen von +20°C bis +150°C durchgeführt.

Es wird bevorzugt bei Normaldruck gearbeitet.

Als Verdünnungsmittel können alle inerten organischen Lösungsmittel, die mit Wasser mischbar sind, verwendet werden. Hierzu zählen Ether wie Tetrahydrofuran, Dioxan; Nitrile, wie Acetonitril; Amide wie Dimethylformamid; Alkohole wie Methanol, Ethanol; Dimethylsulfoxid.

Wie bereits erwähnt lassen sich die neuen Verbindungen der Formel XVIII nach den unter (18) angegebenen verfahren herstellen.

Setzt man bei Verfahren (18) als Alkohol der Formel XXVII 3-Trifluormethyl-4-pyrrolobenzylalkohol ein, läßt sich die Reaktion durch folgendes Schema darstellen:

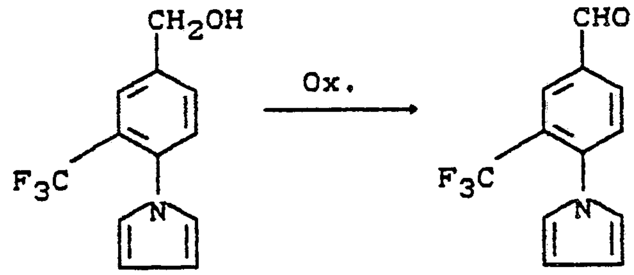


Die Verbindungen der Formel XXVII sind neu. Sie lassen sich nach dem unter (24) angegebenen Verfahren herstellen. Bevorzugt sind Verbindungen der Formel XXVII, in denen $R^1$ und $R^2$ die bei den Verbindungen der Formel I angegebenen bevorzugten Bedeutungen besitzen.

Im einzelnen seien folgende Verbindungen der Formel XXVII genannt:

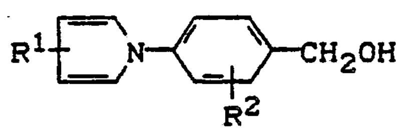

| $R^1$ | $R^2$ |
|---|---|
| H | 3.5-$Cl_2$ |
| 2.5$(CH_3)_2$ | 3.5-$Cl_2$ |
| H | 3-Cl |
| 2.5$(CH_3)_2$ | 3-Cl |
| H | 3-Cl 5-$CF_3$ |
| H | 3-CN |
| 2.5$(CH_3)_2$ | 3-Cl 5-$CF_3$ |

Als Oxidationsmittel zur Durchführung des Verfahrens seien genannt: a) aktiviertes DMSO wie DMSO/Acetanhydrid, DMSO/Thionylchlorid, DMSO/Oxalylchlorid sowie b) Mangandioxid.

Verfahren a) wird durchgeführt, indem man den Alkohol der Formel XIII mit 1-1,5 Äquivalenten des Oxidationsmittels umsetzt.

Die Reaktion wird bei Temperaturen von -70°C bis +25°C durchgeführt.

Es wird bevorzugt bei Normaldruck gearbeitet.

Als Verdünnungsmittel dienen inerte organ. Lösungsmittel, beispielhaft seien genannt:

Gegebenenfalls chlorierte Kohlenwasserstoffe wie Methylenchlorid, Chlorform, Ether wie Diethylether und Tetrahydrofuran.

Verfahren b) wird durchgeführt, indem man den Alkohol der Formel XXVII mit überschüssigem Mangandioxid umsetzt.

Die Reaktion wird bei Temperaturen von +20°C bis +150°C durchgeführt, es wird bevorzugt bei Normaldruck gearbeitet.

Als Verdünnungsmittel werden verwendet: inerte organische Lösungsmittel, insbesondere aliphatische und aromatische gegebenenfalls halogenierte Kohlenstoffe wie Pentan, Hexan, Cyclohexan, Benzol, Toluol, Methylenchlorid, Chloroform, Ether wie Diethylether und Tetrahydrofuran, Ketone wie Aceton und Methylethylketon.

Setzt man bei Verfahren 18 als Verbindung der Formel XXVIII 3-Chlor-4-pyrrolobenzoesäurechlorid ein, läßt sich die Reaktion durch das folgende Schema darstellen:

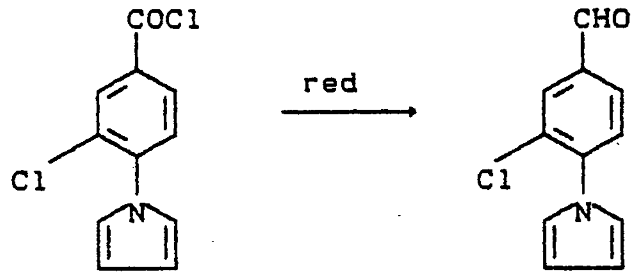


Verbindungen der Formel XXVIII sind neu. Sie lassen sich nach dem unter (26) angegebenen Verfahren herstellen. Bevorzugt seien Verbindungen der Formel XXVIII genannt, in denen $R^1$ und $R^2$ die bei den Verbindungen der Formel I genannten bevorzugten Bedeutungen besitzen.

Im einzelnen seien folgende Verbindungen der Formel XXVIII genannt:

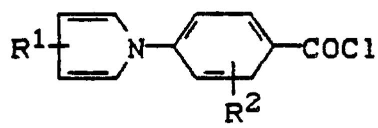

| $R^1$ | $R^2$ |
|---|---|
| H | 3,5-$Cl_2$ |
| 2,5$(CH_3)_2$ | 3,5-$Cl_2$ |
| H | 3-Cl |
| 2,5$(CH_3)_2$ | 3-Cl |
| H | 3-Cl 5-$CF_3$ |
| H | 3-CN |
| 2,5$(CH_3)_2$ | 3-Cl 5-$CF_3$ |

Als Reduktionsmittel dient $H_2$/Katalysator, als Katalysator sei beispielhaft Palladium auf Bariumsulfat genannt.

Das Verfahren wird durchgeführt, indem man durch eine Lösung der Verbindung XXVIII in einem siedenden Verdünnungsmittel nach Zugabe von 5-10 mol-% Katalysator einen Wasserstoffstrom leitet.

Die Reaktion wird bei Temperaturen von 100-200°C durchgeführt, es wird bevorzugt bei Normaldruck gearbeitet.

Als Verdünnungsmittel dienen aliphatische und aromatische Kohlenwasserstoffe. Beispielhaft seien genannt: Hexan, Heptan, Cyclohexan, Benzol, Toluol, Xylole.

Wie bereits erwähnt lassen sich die neuen Verbindungen der Formel XIX nach dem unter 20 angegebenen Verfahren herstellen.

Setzt man bei Verfahren (20) als Verbindung der Formel XXIX 3-Chlor-4-pyrrolocarbethoxyacetophenon ein, läßt sich das Verfahren durch das folgende Schema darstellen:

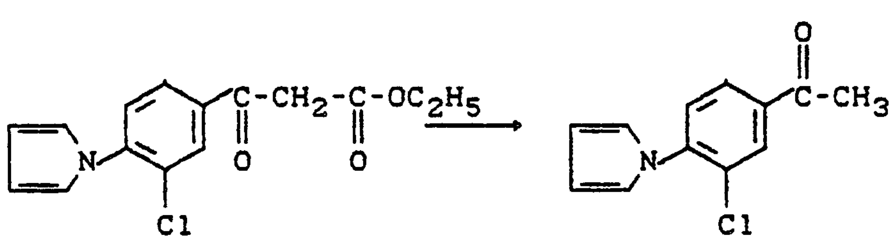

Die Verbindungen der Formel XXIX sind neu. Sie lassen sich nach dem unter (28) beschriebenen Verfahren herstellen. Bevorzugt werden Verbindungen der Formel XIX eingesetzt, in welcher $R^1$ und $R^2$ die bei den Verbindungen der Formel I angegebenen bevorzugten Bedeutungen besitzen und Alk für Methyl oder Ethyl steht.

Im einzelnen seien folgende Verbindungen genannt:

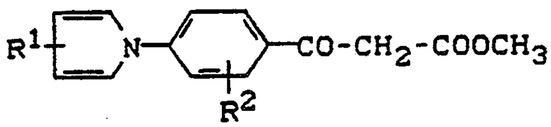

| $R^1$ | $R^2$ |
|---|---|
| H | 3.5-$Cl_2$ |
| 2.5($CH_3$)$_2$ | 3.5-$Cl_2$ |
| H | 3-Cl |
| 2.5($CH_3$)$_2$ | 3-Cl |
| H | 3-Cl 5-$CF_3$ |
| H | 3-CN |
| 2.5($CH_3$)$_2$ | 3-Cl 5-$CF_3$ |

Das Verfahren wird durchgeführt, indem man die Verbindung der Formel XXIX in einem Verdünnungsmittel in Gegenwart eines Überschusses einer Säure oder Base umsetzt.

Die Reaktion wird bei Temperaturen von +20°C bis +150°C durchgeführt.

Es wird bevorzugt bei Normaldruck gearbeitet.

Als Verdünnungsmittel dienen alle inerten organischen Lösungsmittel. Hierzu gehören insbesondere aliphatische und aromatische gegebenenfalls halogenierte Kohlenwasserstoffe wie Pentan, Hexan, Cyclohexan, Benzol, Toluol, Methylenchlorid, Chloroform, Chlorbenzol; Ether wie Diethylether, Tetrahydrofuran, Ethylenglykolmonomethylether, Ethylenglykoldimethylether, Alkohole wie Methanol, Ethanol, sowie Wasser. Bevorzugt wird in Alkohol oder in Wasser gearbeitet.

Als Säuren können alle anorganischen Säuren verwendet werden. Hierzu zählen insbesondere Halogenwasserstoff säuren wie Chlorwasserstoff, des weiteren Schwefelsäure, Phosphorsäure.

Als Basen können alle anorganischen Basen verwendet werden. Hierzu zählen Alkali-und Erdalkalimetallcarbonate wie Natrium-und Kaliumcarbonat sowie Hydroxide wie Natrium-und Kaliumhydroxid.

Wie bereits erwähnt lassen sich die neuen Verbindungen der Formel XXIV nach dem unter (22) angegebenen Verfahren herstellen.

Setzt man bei Verfahren (22) als Aldehyd der Formel XVIII 3-Methyl-4-pyrrolobenzaldehyd ein, läßt sich Verfahren 20 durch das folgende Schema darstellen:

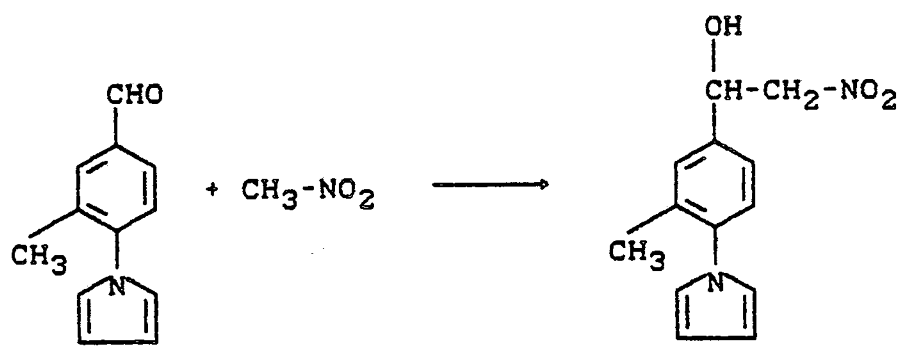

Als Aldehyde der Formel XVIII werden bevorzugt die weiter obengenannten Verbindungen eingesetzt.

Das Verfahren wird durchgeführt, indem man äquivalente Mengen der Verbindung XVIII und Nitromethan in einem Verdünnungsmittel in Gegenwart einer Base umsetzt.

Die Reaktion wird bei Temperaturen von -20°C bis +50°C durchgeführt.

Es wird bevorzugt bei Normaldruck gearbeitet.

Als Verdünnungsmittel dienen alle inerten organischen Lösungsmittel. Hierzu gehören insbesondere Ether wie Diethylether, Tetrahydrofuran; Alkohole wie Methanol, Ethanol.

Als Basen werden verwendet: Alkali-und Erdalkalihydroxide wie Natrium-und Kaliumhydroxid, -alkoholate wie Natrium-und Kaliummethylat, Natrium-und Kaliumethylat.

Wie bereits erwähnt, lassen sich die neuen Verbindungen der Formel XVII nach dem unter (24) angegebenen Verfahren herstellen, indem man entsprechend substituierte Benzoesäuren oder -ester der Formel XXXIII reduziert.

Die Verbindungen der Formel XXXIII sind neu. Sie lassen sich nach dem weiter unten angegebenen Verfahren (30) herstellen. Bevorzugt werden Verbindungen der Formel XXXIII eingesetzt, in welcher $R^1$, $R^2$ die bei den Verbindungen der Formel I genannten bevorzugten Bedeutungen besitzen und $R^9$ für Wasserstoff, Methyl oder Ethyl steht.

Im einzelnen seien folgende Verbindungen der Formel XXXIII genannt:

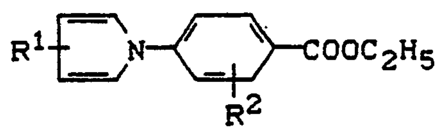

| $R^1$ | $R^2$ |
|---|---|
| H | 3.5-$Cl_2$ |
| 2.5($CH_3$)$_2$ | 3.5-$Cl_2$ |
| H | 3-Cl |
| 2.5($CH_3$)$_2$ | 3-Cl |
| H | 3-Cl 5-$CF_3$ |
| H | 3-CN |
| 2.5($CH_3$)$_2$ | 3-Cl 5-$CF_3$ |

Als Reduktionsmittel dienen
für Ester der Formel XXXIII, komplexe Metallhydride wie z.B. $LiAlH_4$,

für Säuren der Formel XXXIII, Borane wie z.B. Diboran, komplexe Metallhydride, wie z.B. $LiAlH_4$.

Das Verfahren wird durchgeführt, indem man die Verbindungen der Formel XXXIII in einem Verdünnungsmittel mit der 1-4 molaren Menge Reduktionsmittel umsetzt.

Die Reaktion wird bei Temperaturen von -50°C bis +100°C durchgeführt, es wird bevorzugt bei Normaldruck gearbeitet.

Als Verdünnungsmittel dienen:

Ether wie Diethylether, Tetrahydrofuran, Dioxan.

Wie bereits erwähnt lassen sich die neuen Verbindungen der Formel XXXIII nach dem unter (26) genannten Verfahren herstellen.

Setzt man bei Verfahren (26) als Carbonsäure der Formel XXXIII 3-Brom-4-pyrrolobenzoesäure ein, läßt sich die Reaktion durch das folgende Schema darstellen:

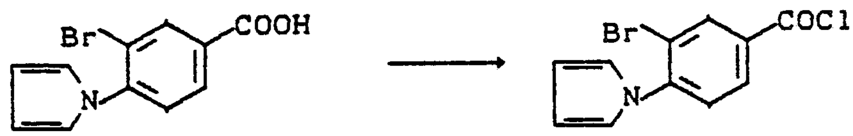

Die Verbindungen der Formel XXXIII sind neu. Sie lassen sich nach dem weiter unten (30) beschriebenen Verfahren herstellen. Es werden bevorzugt die weiter oben angegebenen Verbindungen der Formel XXXIII eingesetzt.

Als Halogenierungsmittel werden anorganische Säurechloride verwendet. Beispielhaft seien genannt: Phosphoroxichlorid, Phosphorpentachlorid, Thionylchlorid.

Die Reaktion wird durchgeführt, indem man eine Verbindung der Formel XXXIII mit 0,5-1,5 Äquivalenten des anorganischen Säurechlorids, gegebenenfalls in einem Verdünnungsmittel, behandelt.

Die Umsetzung wird bei Temperaturen von 20°C bis 100°C durchgeführt, es wird bevorzugt bei Normaldruck gearbeitet.

Als Verdünnungsmittel können alle inerten organischen Lösungsmittel verwendet werden. Hierzu zählen insbesondere aliphatische und aromatische gegebenenfalls halogenierte Kohlenwasserstoffe wie Pentan, Hexan, Cyclohexan, Benzol, Toluol, Methylenchlorid, Chloroform, Chlorbenzol; Ether wie Diethylether, Tetrahydrofuran, Dioxan sowie Phosphoroxichlorid.

Bevorzugt wird ohne Verdünnungsmittel gearbeitet.

Wie bereits erwähnt lassen sich die neuen Verbindungen der Formel XXIX nach dem unter (28) angegebenen Verfahren herstellen.

Setzt man bei Verfahren 28 als Verbindung der Formel XXXIII 4-Pyrrolobenzoesäureisopropylester ein und als Verbindung der Formel XXXVI Essigsäuremethylester ein, läßt sich das Verfahren 28 durch folgendes Formelschema darstellen:

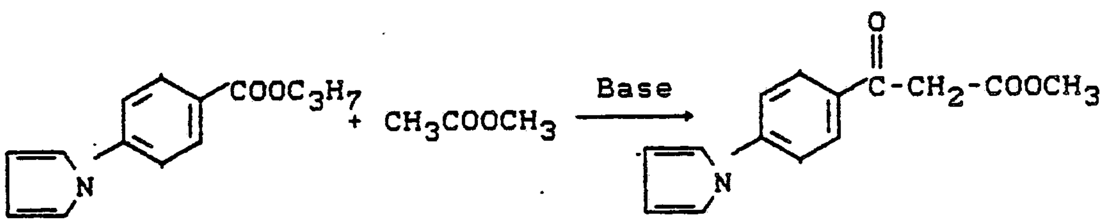


Als Verbindungen der Formel XXXVI werden bevorzugt die weiter oben genannten Verbindungen eingesetzt.

Verbindungen der Formel XXXVI sind bekannt oder lassen sich analog zu bekannten Verfahren herstellen.

Im einzelnen seien folgende Verbindungen der Formel XXXVI genannt:
Essigsäuremethylester, Essigsäureethylester.

Das Verfahren wird durchgeführt, indem man äquivalente Mengen der Verbindungen XXXIII, XXXVI und einer Base in einem Verdünnungsmittel umsetzt.

Die Reaktion wird bei Temperaturen von 0°C bis +150°C durchgeführt.

Es wird bevorzugt bei Normaldruck gearbeitet.

Als Verdünnungsmittel dienen alle inerten organischen Lösungsmittel. Hierzu zählen insbesondere aliphatische und aromatische Kohlenwasserstoffe wie Pentan, Hexan, Heptan, Cyclohexan, Benzol, Toluol; Ether wie Diethylether, Tetrahydrofuran; Alkohole wie Methanol, Ethanol.

Als Basen dienen Alkali-und Erdalkalimetallhydride wie Natrium-und Calciumhydrid, Alkalimetallalkoholate wie Natrium-und Kaliummethylat, Natrium-und Kaliumethylat.

Wie bereits erwähnt lassen sich die neuen Verbindungen der Formel XXXIII nach dem unter (30) genannten Verfahren herstellen.

Die als Ausgangsprodukte für Verfahren (30) dienenden Verbindungen der Formel XXXVII sind bekannt oder lassen sich analog zu bekannten Verfahren herstellen. Bevorzugt sind Verbindungen der Formel XXXVII, in denen $R^2$ die bei den Verbindungen der Formel I angegebenen bevorzugten Bedeutungen besitzt und $R^9$ für Methyl, Ethyl, Propyl oder Wasserstoff steht.

Im einzelnen seien folgende Verbindungen der Formel XXXVII genannt:

#

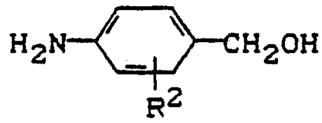

| $R^1$ | $R^2$ |
|---|---|
| H | $3,5-Cl_2$ |
| $2,5(CH_3)_2$ | $3,5-Cl_2$ |
| H | 3-Cl |
| $2,5(CH_3)_2$ | 3-Cl |
| H | 3-Cl $5-CF_3$ |
| H | 3-CN |
| $2,5(CH_3)_2$ | 3-Cl $5-CF_3$ |

Die Wirkstoffe eignen sich bei günstiger Warmblütertoxizität als Mittel zur Leistungsförderung bei Zucht-und Nutztieren. Sie dienen dabei zur Förderung und Beschleunigung des Wachstums, der Milch-und Wollproduktion, sowie zur Verbesserung der Futterverwertung, der Fleischqualität und zur Verschiebung des Fleisch-Fett-Verhältnisses zugunsten von Fleisch.

Darüber hinaus zeigen die erfindungsgemäßen Verbindungen gute antiinflammatorische Wirkung im durch Carrageenan induzierten Pfotenödem-Test an der Ratte.

Sie können somit als Antiphlogistika, Antirheumatika sowie zur Behandlung von Entzündungen und Ödemen eingesetzt werden.

Zu den Nutz-und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß und Salzwasserfische wie z.B. Forellen, Karpfen, Aale, Reptilien.

Die Wirkstoffe werden unabhängig von der Rasse und dem Geschlecht der Tiere während allen Wachstums-und Leistungsphasen der Tiere eingesetzt. Bevorzugt werden die Wirkstoffe während der intensiven Wachstums-und Leistungsphase eingesetzt. Die intensive Wachstums-und Leistungsphase dauert je nach Tierart von einem Monat bis zu 10 Jahren. Besonders wertvoll erweisen sich die Wirkstoffe bei Aufzucht und Haltung von Jung-und Masttieren.

Die Anwendung der Wirkstoffe erfolgt direkt oder in Form von für Tiere geeigneten Zubereitungen enteral oder parentereal. Die enterale Anwendung der Wirkstoffe geschieht z.B. oral in Form von Pulvern, Tabletten, Kapseln, Pasten, Tränken, Granulaten, oral applizirbaren Lösungen, Emulsionen oder Suspensionen, Boli sowie über das Futter oder über das Trinkwasser. Die parenterale Anwendung geschieht z.B. in Form der Injektion (intramuskulär, subcutan, intravenös oder durch Implantate).

Besonders hervorgehoben seien Zubereitungen zur Verabreichung über das Futter oder das Trinkwasser. Dabei können die Wirkstoffe dem Futter direkt oder in Form von Prämixen oder Futterkonzentraten zugesetzt werden.

Zum Futter zählen Einzelfuttermittel pflanzlicher Herkunft wie Heu, Rüben, Getreide und Getreidenebenprodukte, Melasse, Silage, Einzelfuttermittel tierischer Herkunft wie Fleisch, Fette, Milchprodukte, Knochenmehl, Fischprodukte, die Einzelfuttermittel wie Vitamine, Proteine, Zucker, Stärke, Mehle, Aminosäuren z.B. DL-Methionin, Salze wie Kalk und Kochsalz. Zum Futter zählen auch Ergänzungs-, Fertig-und Mischfuttermittel. Diese enthalten Einzelfuttermittel in einer Zusammensetzung, die eine ausgewogene Ernährung hinsichtlich der Energie-und Proteinversorgung sowie der Versorgung mit Vitaminen, Mineralsalzen und Spurenelementen sicherstellt.

Prämixe und Futterkonzentrate sind Mischungen des Wirkstoffs mit Trägerstoffen und gegebenenfalls weiteren Hilfsstoffen. Zu den Trägerstoffen zählen alle Einzelfuttermittel oder Gemische derselben.

Die Wirkstoffe können in den Zubereitungen allein oder in Mischung mit anderen leistungsfördernden Wirkstoffen, mineralischen Futtermitteln, Spurenelement-Verbindungen, Vitaminen, stickstoffhaltigen nicht-Protein-Verbindungen, Farbstoffen, Antioxidantien, Aromastoffen, Emulgatoren, Fließhilfsstoffen,

Konservierungs-und Preßhilfsstoffen vorliegen.

Andere leistungsfördernde Wirkstoffe sind z.B. Antibiotika wie Tylosin und Virginiamycin.

Mineralische Futtermittel sind z.B. Dicalciumphosphat, Magnesiumoxid, Natriumchlorid.

Spurenelement-Verbindungen sind z.B. Eisenfumarat, Natriumjodid, Kobaltchlorid, Kupfersulfat, Zinkoxid, Selenverbindungen .

Vitamine sind z.B. Vitamin A, Vitamin $D_3$, Vitamin E.

Stickstoffhaltige nicht-Protein-Verbindungen sind z.B. Biuret, Harnstoff.

Farbstoffe sind z.B. Carotinoide wie Canthaxidin, Zeaxanthin, Capsanthin oder alle Farbstoffe, die zur Färbung von Lebensmitteln zugelassen sind.

Antioxidantien sind z.B. Ethoxyquin, Butylhydroxy-Toluol, Ascorbinsäure.

Aromastoffe sind z.B. Vanillin.

Emulgatoren sind z.B. Ester der Milchsäure, Lecithin.

Fließhilfsstoffe sind z.B. Natriumstearat, Calciumstearat, Kieselsäuren, Bentonite, Ligninsulfonate.

Konservierungsstoffe sind z.B. Propionsäure, Calciumpropionat, Sorbinsäure, Ascorbinsäure.

Preßhilfsstoffe sind z.B. Ligninsulfonate, Celluloseether.

Die Konzentration der Wirkstoffe im Futter beträgt normalerweise etwa 0,001-500 ppm, bevorzugt 0,1-50 ppm.

Die Konzentration der Wirkstoffe in den Prämixen oder Futterkonzentraten beträgt ca. 0,5 bis 50 Gewichtsprozent, bevorzugt 1 bis 20 Gewichtsprozent.

Die Menge der Wirkstoffe, die den Tieren zur Erreichung des gewünschten Effektes verabreicht wird, kann wegen der günstigen Eigenschaften der Wirkstoffe weitgehend variiert werden. Sie liegt vorzugsweise bei etwa 0,001 bis 50 mg/kg insbesondere 0,01 bis 5 mg/kg Körpergewicht pro Tag. Die passende Menge des Wirkstoffs sowie die passende Dauer der Verabreichung hängen insbesondere von der Art, dem Alter, dem Geschlecht, dem Gesundheitszustand und der Art der Haltung und Fütterung der Tiere ab und sind durch jeden Fachmann leicht zu ermitteln.

Die Wirkstoffe werden den Tieren nach den üblichen Methoden verabreicht. Die Art der Verarbreichung hängt insbesondere von der Art, dem Verhalten und dem Gesundheitszustand der Tiere ab.

Die Wirkstoffe können einmalig verabreicht werden. Die Wirkstoffe können aber auch während der ganzen oder während eines Teils der Wachstums-und Leistungsphase temporär oder kontinuierlich verabreicht werden. Bei kontinuierlicher Verabreichung kann die Anwendung ein-oder mehrmals täglich in regelmäßigen oder unregelmäßigen Abständen erfolgen.

Beispiel für die Zusammensetzung eines Kükenaufzuchtfutters, das erfindungsgemäßen Wirkstoff enthält:

200 g Weizen, 340 g Mais, 361 g Sojaschrot, 60 g Rindertalg, 15 g Dicalciumphosphat, 10 g Calciumcarbonat, 4 g jodiertes Kochsalz, 7,5 g Vitamin-Mineral-Mischung der unten angegebenen Zusammensetzung und 2,5 g Wirkstoff-Prämix der unten angegebenen Zusammensetzung ergeben nach sorgfältigem Mischen 1 kg Futter.

In einem kg Vitamin-Mineral-Mischung sind enthalten: 600 I.E. Vitamin A, 100 I.E. Vitamin $D_3$, 10 mg Vitamin E, 1 mg Vitamin $K_3$, 3 mg Riboflavin, 2 mg Pyridoxin, 20 mcg Vitamin $B_{12}$, 5 mg Calciumpantothenat, 30 mg Nikotinsäure, 200 mg Cholinchlorid, 200 mg Mn $SO_4$ x $H_2O$, 140 mg Zn $SO_4$ x 7 $H_2O$, 100 mg Fe $SO_4$ x 7 $H_2O$ und 20 mg Cu $SO_4$ x 5 $H_2O$ in Getreidemehl als Trägerstoff.

1 kg Wirkstoff-Prämix enthalten 100 g Wirkstoff, 900 g Weizenmehl.

Beispiel für die Zusammensetzung eines Schweineaufzuchtfutters, das erfindungsgemäßen Wirkstoff enthält:

630 g Futtergetreideschrot (zusammengesetzt aus 200 g Mais, 150 g Gerste-, 150 g Hafer-und 130 g Weizenschrot), 80 g Fischmehl, 60 g Sojaschrot, 60 g Tapiokamehl, 38 g Bierhefe, 50 g Vitamin-Mineral-Mischung (Zusammensetzung wie beim Kükenfutter) 30 g Leinkuchenmehl, 30 g Maiskleberfutter, 10 g Sojaöl, 10 g Zuckerrohrmelasse und 2 g Wirkstoff-Prämix der unten angegebenen Zusammensetzung ergeben nach sorgfältigem Mischen 1 kg Futter.

1 kg Wirkstoff Prämix enthält 200 g Wirkstoff, 20 g Pflanzenöl und 780 g Calciumcarbonatpulver.

Beispiel für die Zusammensetzung eines Futters für Rinder, das den erfindungsgemäßen Wirkstoff enthält:

69,95 % Futtergetreideschrot, 10 % gemahlene Maiskolben, 8 % Sojabohnenmehl, 5 % Luzernemehl, 5 % Melasse, 0,6 % Harnstoff, 0,5 % Calciumphosphat, 0,5 % Calciumcarbonat, 0,3 % Kochsalz und 0,15 % Vitamin-Mineral-Mischung. Die Vitamin-Mineral-Mischung enthält pro kg 70.000 i.E. Vitamin A, 70.000 i:E.

Vitamin $D_3$, 100 mg Vitamin E, 50 mg Mn $SO_4$ x $H_2O$, 30 mg Zn $SO_4$ x $7H_2O$ in Getreidemehl als Trägerstoff.

Der Wirkstoff wird der Vitamin-Mineral-Mischung in der erforderlichen Menge beigemischt und diese anschließend sorgfältig mit den übrigen Bestandteilen vermischt.

Beispiel A

Ratten-Fütterungsversuch

Weibliche Laborraten 90-110 g schwer vom Typ SPF Wistar (Züchtung Hagemann) werden ad lib mit Standard Rattenfutter, das mit der gewünschten Menge Wirkstoff versetzt ist, gefüttert. Jeder Versuchsansatz wird mit Futter der identischen Charge durchgeführt, so daß Unterschiede in der Zusammensetzung des Futters die Vergleichbarkeit der Ergebnisse nicht beeinträchtigen können.

Die Ratten erhalten Wasser ad lib.

Jeweils 12 Ratten bilden eine Versuchsgruppe und werden mit Futter, das mit der gewünschten Menge Wirkstoff versetzt ist gefüttert. Eine Kontrollgruppe erhält Futter ohne Wirkstoff. Das durchschnittliche Körpergewicht sowie die Streuung in den Körpergewichten der Ratten ist in jeder Versuchsgruppe gleich, so daß eine Vergleichbarkeit der Versuchsgruppen untereinander gewährleistet ist.

Während des 13-tägigen Versuchs wird die Gewichtszunahme bestimmt.

Es werden die aus der Tabelle ersichtlichen Ergebnisse erhalten:

Tabelle

Ratten-Fütterungsversuch

| Wirkstoff Bsp.-Nr. | Dosis 25 ppm | Gewichtszunahme nach 13 Tagen |
|---|---|---|
| Kontrolle ohne Wirkstoff | | 100 |
| 1 | | 135 |
| 2 | | 130 |
| 10 | | 131 |
| 12 | | 131 |
| 12.7 | | 137 |
| 12.9 | | 129 |
| 11.1 | | 120 |
| 8 | | 125 |
| 12.8 | | 137 |
| 12.14 | | 115 |
| 12.12 | | 122 |
| 13 | | 125 |
| 13.1 | | 145 |

## Herstellungsbeispiele

### Beispiel 1

2-(3,5-Dichlor-4-pyrrolophenyl)-2-hydroxy-N-tert.-butyl-1-ethylamin

10 g 2-(3,5-Dichlor-4-amino-phenyl)-2-hydroxy-N-tert.-butyl-1-ethylamin werden mit 5,3 g 2,5-Dimethoxytetrahydrofuran in 50 ml Essigsäure 1 Stunde unter Rückfluß erhitzt. Nach dem Abkühlen gießt man auf 500 ml Wasser, macht mit konzentrierter Ammoniaklösung alkalisch, extrahiert 2 x mit je 50 ml Essigester und trennt die organischen Phasen ab und trocknet sie über Natriumsulfat. Man dampft ein und erhält nach chromatographischer Reinigung/Kieselgelsäule ($HCCl_3$:$CH_3OH$:$NH_3$ = 20:3:0,3) 10 g vom Fp.: 125°C.

### Beispiel 2

2-[3,5-Dichlor-4-(2′,5′-dimethylpyrrolo)-phenyl]-2-hydroxy-N-tert.-butyl-1-ethylamin

Eine Lösung von 15 g 2-(3,5-Dichlor-4-amino-phenyl)-2-hydroxy-N-tert.-butyl-1-ethylamin und 10,3 g 2,5-Hexandion in 75 ml Essigsäure wurde 12 Stunden unter Rückfluß erhitzt. Man erhielt nach der in Beispiel 1 beschriebenen Aufarbeitung 10,2 g vom Fp.: 95°C.

Anstelle von 2,5-Hexandion kann man 2,5-Dimethyl-2,5-dimethoxy-tetrahydrofuran verwenden und erhält die o.g. Verbindung in einer Ausbeute von 13 g. Fp.: 95°C.

### Beispiel 2a

Nach der in Beispiel 1 und 2 beschriebenen Reaktion erhielt man folgende Verbindungen, die in $^1H$-NMR charakterisiert wurden.

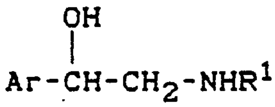

| Bsp.Nr. | Ar | $R^1$ | Fp° C |
|---|---|---|---|
| 3 | 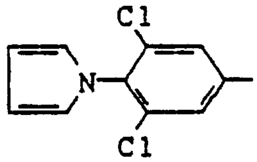 | $CH(CH_3)_2$ | 120 |
| 4 | 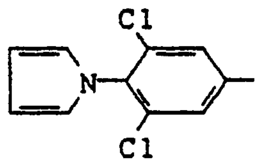 | 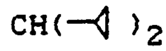 | Öl |
| 5 | 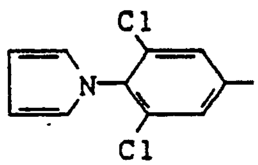 | 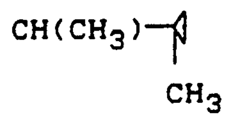 | 100 |
| 6 | 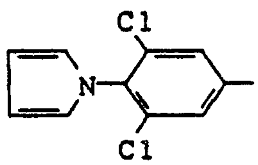 | 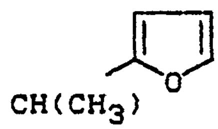 | 120-125 |
| 7 | 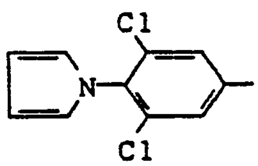 | 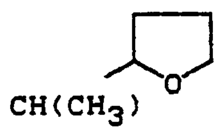 | Isomer A Öl<br>Isomer B Öl |

| Bsp.Nr. | Ar | $R^1$ | Fp° C |
|---|---|---|---|
| 8 | 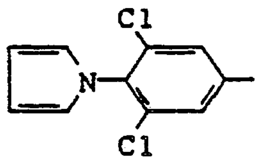 | $CH(CH_3)-$ 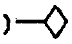 | 110 |
| 9 | 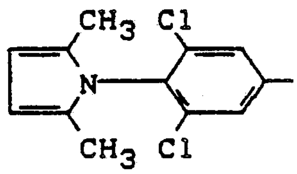 | $CH(CH_3)-$ 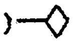 | Öl |
| 10 | 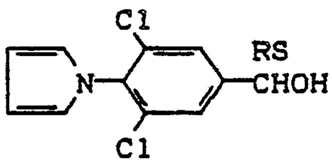 | RS R $-CHOH-CH_2-NH-CH(CH_3)-$ 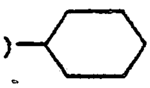 $[\alpha]^{20}_{589} = -7,4$ (Methanol, c=0,74) | 86- 90 |
| 11.1 | 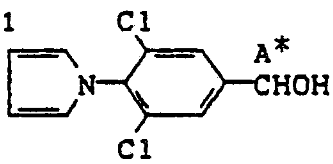 | A* R $-CHOH-CH_2-NH-CH(CH_3)-$ 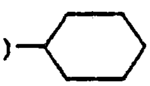 $[\alpha]^{20}_{589} = -25,1$ (Methanol, c=0,77) | 114 |
| | * = Diastereomer A | | |
| 11.2 | 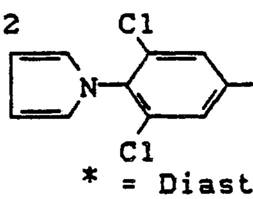 | B* R $-CHOH-CH_2-NH-CH(CH_3)-$ 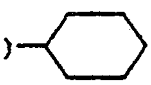 | |
| | * = Diastereomer B | | |
| 12 | 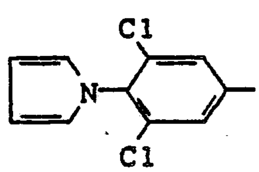 | $CH(CH_3)-$ 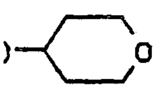 | Öl |

| Bsp.Nr. | Ar | $R^1$ | Fp° C |
|---|---|---|---|
| 12.1 | 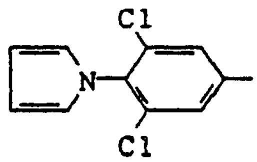 | $CH(CH_3)_3$ | 98-100 |
| 12.2 | 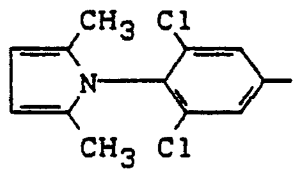 | $CH(CH_3)_3$ | Öl |
| 12.3 | 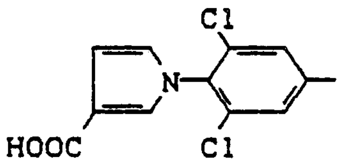 | $C(CH_3)_2$ | 260 |
| 12.4 | 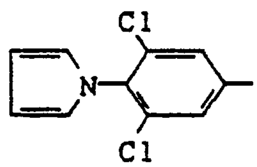 | 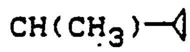 | 97 |
| 12.5 | 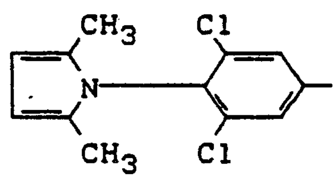 | 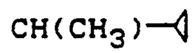 | Öl |
| 12.6 | 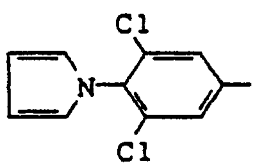 | 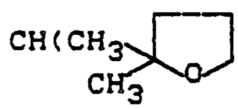 | 65 |
| 12.7 | 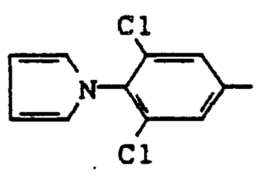 | $CH(CH_3)-CH_2OCH_3$ | 75 |
| 12.7.1 | | Diastereomer A | 72 |

| Bsp.Nr. | Ar | $R^1$ | Fp° C |
|---|---|---|---|
| 12.8 | 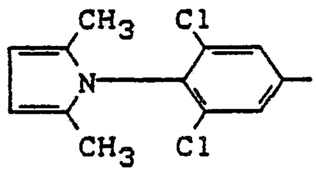 | $CH(CH_3)-CH_2-OCH_3$ | Öl |
| 12.9 | 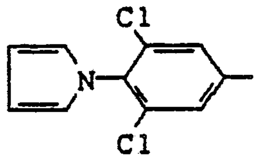 | $CH(CH_3)-$ 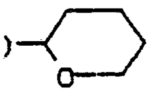 | Öl |
| 12.10 | 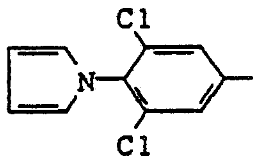 | $CH(CH_3)-C(CH_3)_2-OCH_3$ | 75 |
| 12.11 | 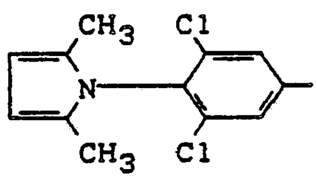 | $CH(CH_3)_2-OCH_3$ | Öl |
| 12.12 | 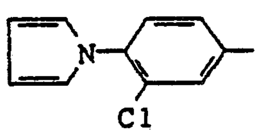 | $C(CH_3)_3$ | 74-76 |
| 12.13 | 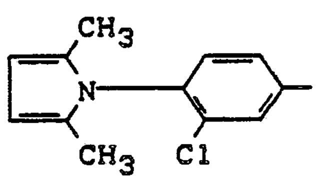 | $C(CH_3)_3$ | Öl |
| 12.14 | 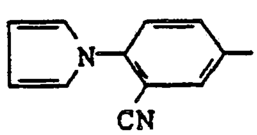 | $CH(CH_3)_2$ | 115-118 |

| Bsp.Nr. | Ar | $R^1$ | Fp° C |
|---|---|---|---|
| 12.15 | 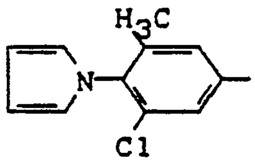 | $C(CH_3)_3$ | 102 |
| 12.16 | 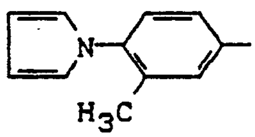 | $C(CH_3)_3$ | Öl |
| 12.17 | 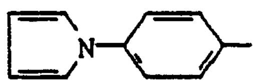 | $C(CH_3)_3$ | 113 |

Beispiel 13

2-(3,5-Dichlor-4-pyrrolo-phenyl)-2-(2,2-dimethylpropyldimethyl-silyloxy)-N-tert.-butyl-ethylamin

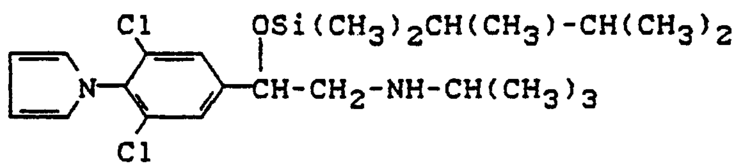

1,38 g (0,02 Mol) Imidazol werden in 15 ml abs. DMF mit 3,27 g (0,01 Mol) 2-(3,5-Dichlor-4-pyrrolo-phenyl)-2-hydroxy-N-tert.-butyl-ethylamin (Beispiel 1) versetzt. Bei 0 bis 5°C werden nun 1,8 g Dimethyl-1,2-dimethylpropylsilylchlorid zugegeben. Es wird 2 Stunden unter Eiskühlung gerührt, eingeengt, in Toluol/Wasser aufgenommen, getrennt, die Toluolphase 4 x mit Wasser gewaschen, getrocknet, eingeengt und an der Ölpumpe von Lösemittelresten befreit. Man erhält 4,8 g eines nahezu farblosen Öles.

13.1

In entsprechender Arbeitsweise erhält man mit Dimethyl-tert.-butyl-silylchlorid

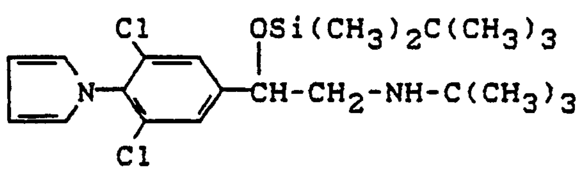

als farbloses Öl.

Beispiel 14

5-(3,5-Dichlor-4-pyrrolo-phenyl)-3-tert.-butyl-2-oxazolidinon

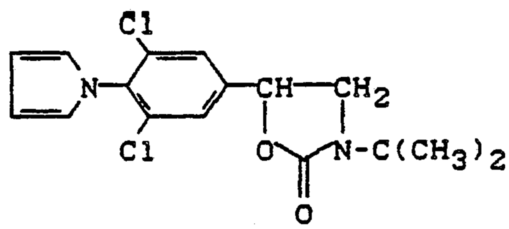

Zu 3,27 g (0,01 Mol) 2-(3,5-Dichlor-4-pyrrolo-phenyl)-2-hydroxy-N-tert.-butyl-ethylamin, gelöst in 30 ml Methylenchlorid und 3 g Triethylamin tropft man bei -5°C eine Lösung von 1 g $COCl_2$ in 20 ml Methylenchlorid innerhalb von 20 Minuten. Man rührt 30 Minuten bei -5°C und 1 Stunde bei Raumtemperatur, dampft ein im Vakuum und chromatographiert den Rückstand über eine Kieselgelsäule mit n-Heptan, Methylenchlorid = 1:1. Ausbeute 1,5 g.

Beispiel 15

5-(3,5-Dichlor-4-pyrrolo-phenyl)-3-tert.-butyl-oxazolidin

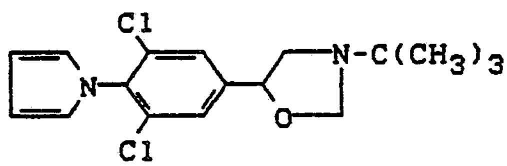

3,27 g (0,01 Mol) 2-(3,5-Dichlor-4-pyrrolo-phenyl)-2-hydroxy-N-tert.-butyl-ethylamin gelöst in 60 ml Toluol erhitzt man mit 18 ml Formaldehyd (38 %ig) unter Zusatz von 2 mg p-Toluolsulfonsäure am Wasserabscheider. Wenn sich kein Wasser mehr abscheidet (ca. 1 Stunde) kühlt man ab, wäscht 2 mal mit 10 %iger Natronlauge, dann mit Wasser und trocknet über $Na_2SO_4$. Nach dem Abdampfen chromatographiert man über ein Kieselgelsäule (Hexan, Methylenchlorid = 1:1) und erhält 2,5 g eines Öles.

Beispiel 16

2-(3,5-Dichlor-4-pyrrolo-phenyl)-2-acetoxy-N-tert.-butyl-ethylamin

Zu 3,27 g (0,01 Mol) 2-(3,5-Dichlor-4-pyrrolo-phenyl)-2-hydroxy-N-tert.-butyl-ethylamin gelöst in 50 ml $CHCl_3$ fügt man 3 g Triethylamin, tropft 1,1 g (0,011 Mol) Acetanhydrid bei -5°C und erwärmt 2 Stunden auf Zimmertemperatur. Nach beendeter Umsetzung (Kontrolle durch DC) dampft man ein im Vakuum, nimmt in Essigester auf, wäscht mit Natriumbicarbonatlösung, dann mit gesättigter Kochsalzlösung, dampft die organische Phase ein und chromatographiert über eine Kieselgelsäule (Hexan-Essigester = 2:1). Ausbeute: 2,8 g.

Die für die Beispiele 11.1 und 11.2 verwendeten Ausgangsverbindungen wurden auf folgendem Wege erhalten:

10,9 g (29,8 mMol) 4-Amino-3,5-dichlor-ω-[(R)-1-cyclohexyl-ethylamino]-acetophenon-hydrochlorid, F 226°C (erhalten aus 4-Amino-3,5-dichlor-ω-brom-acetophenon durch Umsetzung mit (R)-1-Cyclohexyl-ethylamin werden in 75 ml Methanol und 18 ml Wasser gelöst und bei einem pH-Wert zwischen 2 und 7 mit einer Lösung von 2,5 g Natriumborhydrid in 8 ml Wasser tropfenweise versetzt.

Es wurde auf pH 9 eingestellt, eingeengt und mit Essigester extrahiert. Die Essigesterphase wird mit Wasser gewaschen, getrocknet und eingeengt. Das kristalline Produkt wird mit Heptan verrührt, abgesaugt und mit Heptan gewaschen. Man erhält 6,5 g 2-(3,5-Dichlor-4-amino-phenyl)-2-hydroxy-N-[(R)-1-cyclohexyl-ethyl]-ethylamin, F 110 bis 116°C. Durch fraktionierte Umkristallisation aus Acetonitril werden die beiden Diastereomeren getrennt:

Physikalische Daten:
Diastereomer 11.1: Schmelzpunkt: 142°C
$[\alpha]^{20}_{589}$ = -22 (c = 0,69, Methanol)
Diastereomer 11.2: Schmelzpunkt: 108-111°C
$[\alpha]^{20}$ = -27,26 (c = 0,8576, Methanol)

In entsprechender Weise erhält man aus 4-Amino-3,5-dichlor-ω-[(S)-1-cyclohexyl-ethylamino]-acetophenon das 2-(3,5-Dichlor-4-amino-phenyl)-2-hydroxy-N-[(S)-1-cyclohexyl-ethyl]-ethylamin, das durch fraktionierte Kristallisation in die Diastereomeren C und D aufgetrennt wird.

In entsprechender Weise erhält man aus 4-Amino-3,5-dichlor-ω-(cyclohexylethylamino)-acetophenon ein Diastereomerengemisch von 2-(3,5-Dichlor-4-aminophenyl)-2-hydroxy-N-(1-cyclohexyl-ethyl)-ethylamin.

## Ansprüche

1. Pyrrolophenylalkanolamine der Formel I und ihre Derivate

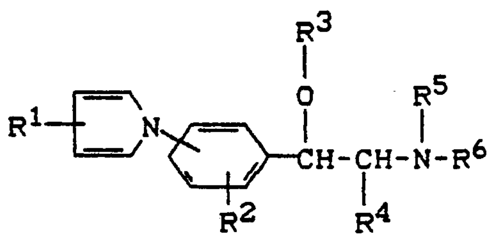

I

in welcher
$R^1$ für einen oder mehrere gleiche oder verschiedene Reste aus der Gruppe Wasserstoff, Halogen, Cyano, Formyl, Nitro, Carboxyl, Carbalkoxyalkyl, Alkoxycarboxyalkyl, Alkylcarbonyl, Alkoxycarbonyl, Aminocarbonyl, Alkylaminocarbonyl, Dialkylaminocarbonyl, Alkyl, Alkoxy, Alkenoxy, Halogenalkyl, Halogenalkoxy, Alkylthio, Halogenalkylthio, Hydroxyalkyl, Cyanoalkyl, Alkoxyalkyl, Alkylthioalkyl, Alkylcarbonylalkoxy steht,
$R^2$ für einen oder mehrere gleiche oder verschiedene Reste aus der Gruppe Wasserstoff, Hydroxy, Halogen, Cyano, Nitro, Alkyl, Alkoxy, Alkylthio, Halogenalkyl, Halogenalkoxy, Halogenalkylthio, Cyanoalkyl, Alkoxyalkyl, Hydroxyalkyl, Alkoxycarbonyl, Aminocarbonyl, Mono-, Dialkylaminocarbonyl steht,
$R^3$ für Wasserstoff, Acyl oder Trialkylsilyl steht,
$R^4$ für Wasserstoff oder Alkyl steht,
$R^5$ für Wasserstoff gemeinsam mit $R^3$ für folgende Reste steht,

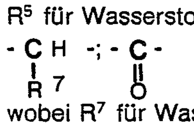

wobei $R^7$ für Wasserstoff oder Alkyl steht,
$R^6$ für verzweigtes oder cyclisches Alkyl steht, die gegebenenfalls substituiert sind.

2. Verfahren zur Herstellung der neuen Pyrrolophenylalkanolamine der Formel I und ihrer Derivate

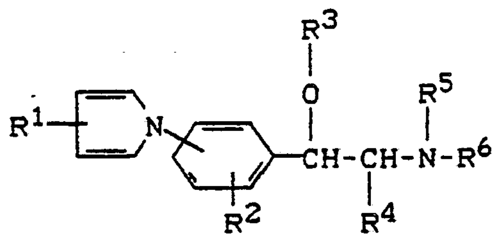

I

in welcher
$R^1$ für einen oder mehrere gleiche oder verschiedene Reste aus der Gruppe Wasserstoff, Halogen, Cyano, Formyl, Nitro, Carboxyl, Carbalkoxyalkyl, Alkoxycarboxyalkyl, Alkylcarbonyl, Alkoxycarbonyl, Aminocarbonyl, Alkylaminocarbonyl, Dialkylaminocarbonyl, Alkyl, Alkoxy, Alkenoxy, Halogenalkyl, Halogenalkoxy, Alkylthio, Halogenalkylthio, Hydroxyalkyl, Cyanoalkyl, Alkoxyalkyl, Alkylthioalkyl, Alkylcarbonylalkoxy steht,
$R^2$ für einen oder mehrere gleiche oder verschiedene Reste aus der Gruppe Wasserstoff, Hydroxy, Halogen, Cyano, Nitro, Alkyl, Alkoxy, Alkylthio, Halogenalkyl, Halogenalkoxy, Halogenalkylthio, Cyanoalkyl, Alkoxyalkyl, Hydroxyalkyl, Alkoxycarbonyl, Aminocarbonyl, Mono-, Dialkylaminocarbonyl steht,
$R^3$ für Wasserstoff, Acyl oder Trialkylsilyl steht,
$R^4$ für Wasserstoff oder Alkyl steht,
$R^5$ für Wasserstoff oder gemeinsam mit $R^3$ für folgende Reste steht,

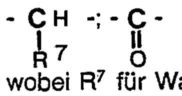

wobei $R^7$ für Wasserstoff oder Alkyl steht,
$R^6$ für verzweigtes oder cyclisches Alkyl steht, die gegebenenfalls substituiert sind,
dadurch gekennzeichnet, daß man Verbindungen der Formel II

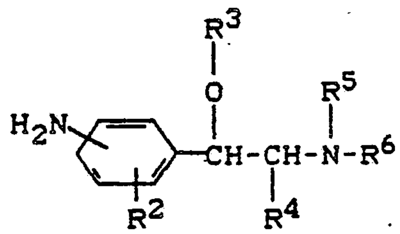

II

in welcher
$R^2$, $R^3$, $R^4$, $R^5$, $R^6$ die oben angegebene Bedeutung haben,
a) mit 1,4-Dicarbonylverbindungen der Formel III

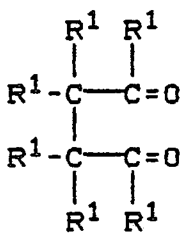

III

oder deren Monoacetale, Diacetale oder cyclische Acetale der Formel IIa, IIIb oder IIIc

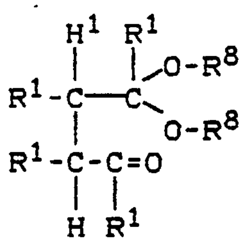 ; 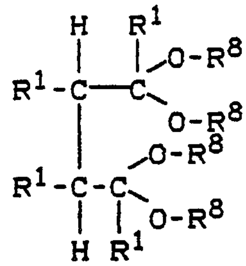

IIIa IIIb

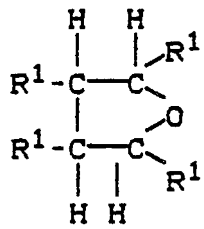

IIIc

in welchen
$R^1$ die oben angegebene Bedeutung hat,
$R^8$ für Wasserstoff, Alkyl oder Aryl steht,
umsetzt oder

b) mit Dihalogenalkenen der Formel IV

Hal-CH = C = CH-$CH_2$-Hal IV

in welcher
Hal für Halogen steht
umsetzt oder

c) mit Epoxybutanen der Formel V

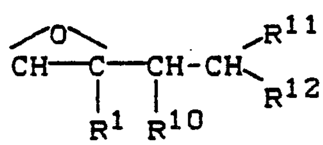

V

in welcher
$R^1$ die oben angegebene Bedeutung hat,
$R^{10}$ für Wasserstoff oder Brom steht,
$R^{11}$ für Wasserstoff und
$R^{12}$ für Brom steht oder
$R^{11}$ und $R^{12}$ für Alkoxy oder Aryloxy stehen,
umsetzt oder

d) mit Halogencrotonaldehyd der Formel VI

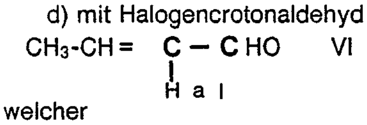 VI

in welcher
Hal für Halogen steht,
umsetzt oder

e) indem man Verbindungen der Formel VII

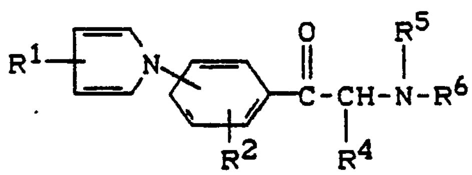

VII

in welcher
$R^1$, $R^2$, $R^4$, $R^5$, $R^6$ die oben angegebene Bedeutung haben,
reduziert oder

f) indem man Verbindungen der Formel VIII

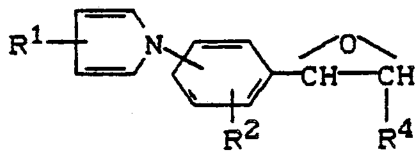

VIII

in welcher
$R^1$, $R^2$, $R^4$ die oben angegebene Bedeutung haben,
mit Aminen der Formel IX

$HNR^5R^6$ IX

in welcher
$R^5$ und $R^6$ die oben angegebene Bedeutung haben,
umsetzt oder

g) indem man Verbindungen der Formel X

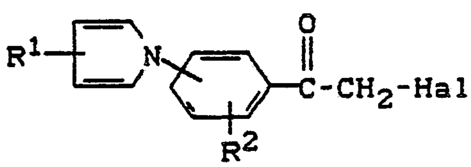

X

in welcher
$R^1$ und $R^2$ die oben angegebene Bedeutung haben und
Hal für Halogen steht,
mit Aminen der Formel IX umsetzt und anschließend die Carbonylgruppe reduziert oder

h) indem man Verbindungen der Formel XI

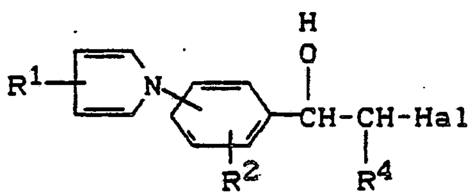

XI

in welcher
$R^1$, $R^2$, $R^4$ die oben angegebene Bedeutung haben,
Hal für Halogen steht,
mit Aminen der Formel IX umsetzt oder

i) indem man für den Fall, daß in Formel I der Rest $R^5$ für Wasserstoff steht, Verbindungen der Formel XII

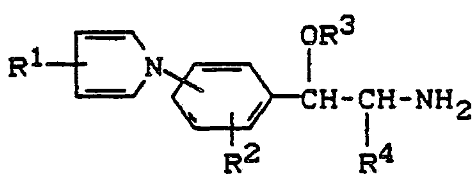

XII

in welcher
$R^1$ bis $R^4$ die oben angegebene Bedeutung haben,

mit Ketonen der Formel XIII

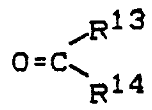

XIII

in welcher
$R^{13}$ für gegebenenfalls substituiertes Alkyl oder Cycloalkyl steht,
$R^{14}$ für gegebenenfalls substituiertes Alkyl, Cycloalkyl oder Heterocyclyl steht,
$R^{13}$ und $R^{14}$ gemeinsam mit dem angrenzenden C-Atom für einen gegebenenfalls substituierten aliphatischen Ring stehen,
unter reduzierenden Bedingungen umsetzt oder

j) indem man Verbindungen der Formel XIV

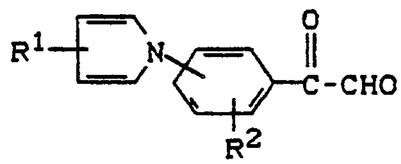

XIV

in welcher
$R^1$ und $R^2$ die oben angegebenen Bedeutungen haben,
mit Aminen der Formel IX unter reduzierenden Bedingungen umsetzt oder

k) indem man für den Fall, daß in Formel I $R^3$ und $R^4$ für Wasserstoff stehen, Verbindungen der Formel XV

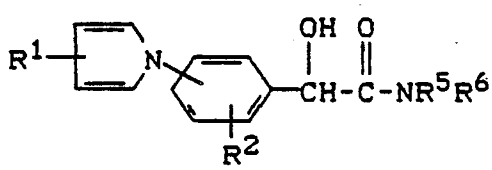

XV

in welcher
$R^1$, $R^2$, $R^5$, $R^6$ die oben angegebenen Bedeutungen haben,
reduziert oder

l) indem man für den Fall, daß $R^3$ für Acyl steht, Verbindungen der Formel I in welcher $R^3$ für Wasserstoff steht, mit Acylierungsmitteln in Gegenwart von Basen umsetzt oder

m) indem man für den Fall, daß $R^3$ für Trialkylsilyl steht, Verbindungen der Formel I in welcher $R^3$ für Wasserstoff steht,
mit Silylierungsmitteln der Formel XVI

$Z\text{-}Si(R^{15})_3$ XVI

in welcher
Z für Halogen, CN, $OSO_2$, $CF_3$, $O\text{-}Si(Alkyl)_3$ oder $O\text{-}SO_2\text{-}OSi(Alkyl)_3$ steht und
$R^{15}$ für gleiche oder verschiedene Alkylreste steht,
umsetzt oder

n) indem man für den Fall, daß $R^3$ und $R^5$ gemeinsam für den Rest

- C -
||
O

stehen, Verbindungen der Formel I in welcher $R^3$ und $R^5$ für Wasserstoff stehen, mit Phosgen oder phosgenabspaltenden Mitteln umsetzt oder

o) indem man für den Fall, daß $R^3$ und $R^5$ gemeinsam für den Rest

- CH -
|
$R^7$

stehen, Verbindungen der Formel I in welcher $R^3$ und $R^5$ für Wasserstoff stehen, mit Aldehyden der Formel XVII

$R^7$- =O XVII

in welcher
$R^7$ die oben angegebene Bedeutung hat,
in Gegenwart wasserentziehender Mittel oder Reaktionsbedingungen umsetzt, oder

p) indem man Verbindungen der Formel XII, in der $R^1$ - $R^4$ die oben angegebene Bedeutung haben, mit Halogenverbindungen der Formel XXXVIII

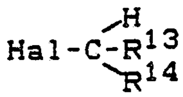

XXXVIII

in welcher
$R^{13}$ und $R^{14}$ die bei Verfahren 2i, bei den Verbindungen der Formel XIII angegebenen Bedeutungen haben und
Hal für Chlor oder Brom steht,
umsetzt.

3. Mittel zur Leistungsförderung von Tieren gekennzeichnet durch einen Gehalt an Pyrrolophenylalkanolaminen der Formel I gemäß Anspruch 1.

4. Tierfutter, Trinkwasser für Tiere, Zusätze für Tierfutter und Trinkwasser für Tiere, gekennzeichnet durch einen Gehalt an Pyrrolophenylalkanolaminen der Formel I gemäß Anspruch 1.

5. Verwendung von Pyrrolophenylalkanolaminen der Formel I gemäß Anspruch 1 zur Leistungsförderung von Tieren.

6. Verfahren zur Herstellung von Mitteln zur Leistungsförderung von Tieren, dadurch gekennzeichnet, daß man Pyrrolophenylalkanolamine der Formel I gemäß Anspruch 1 mit Streck-und/oder Verdünnungsmitteln vermischt.

7. Verfahren zur Herstellung von Tierfutter, Trinkwasser für Tiere oder Zusätze für Tierfutter und Trinkwasser für Tiere, dadurch gekennzeichnet, daß man Pyrrolophenylalkanolamine der Formel I gemäß Anspruch 1 mit Futtermitteln oder Trinkwasser und gegebenenfalls weiteren Hilfsstoffen vermischt.

8. Verwendung von Pyrrolophenylalkanolamine der Formel I gemäß Anspruch 1 zur Herstellung von Mitteln zur Leistungsförderung bei Tieren.